# EUROPEAN PATENT APPLICATION

(11) **EP 2 388 567 A1**
(43) Date of publication of application: **23.11.2011**
(21) Application number: 11171195.8
(22) Date of filing: 28.03.2006
(51) Int. Cl.: G01N 1/30, B01L 3/00, A61B 10/02

(54) **Supporting solution for cytological diagnosis**

(30) Priority: 28.03.2005 KR 20050025668; 28.03.2005 KR 20050025667; 28.03.2005 KR 20050025666; 18.04.2005 KR 20050032078
(62) Divisional of application: 06732717.1
(71) Applicant: Medimex Co., Ltd., Jungwon-gu Seongnam-city, Kyunggi-do 462-729 (KR)
(72) Inventor: Park, Chang Soo, Seongnam-city (KR); Moon, Sang Ho, Seongnam-city (KR)
(74) Representative: Pulieri, Gianluca Antonio

(57) **Abstract**

Disclosed are a tumor screening system, which operates separation of cells from a vial containing a solution mixed with the cells and collection of the cells on a slide using an automatic unit, a collection vial for liquid based cytology serving to simultaneously store a solution containing cells and pour the solution from out of tumor screening system, a brush for liquid based cytology of cervix carcinoma, which collects cells from a woman's uterus, and a supporting solution for cytodiagnosis, which preserves the collected cells.

## Description

### [Technical Field]

The present invention relates to a tumor screening system, a collection vial for liquid based cytology, a brush for liquid based cytology of cervix carcinoma, and a supporting solution for cytodiagnosis. The tumor screening system operates separation of cells from a vial containing a solution mixed with the cells and collection of the cells on a slide using an automatic unit, and the collection vial for liquid based cytology serves to simultaneously store a solution containing cells and pour the solution out of the tumor screening system. The brush for liquid based cytology of cervix carcinoma facilitates the collection of cells from a woman's uterus. The supporting solution for cytodiagnosis comprises a buffer agent for storing collected cells under the condition that the collected cells are maintained in a designated pH range, alcohol for suppressing the protein degeneration of the cells to maintain the pathological structures of the cells, an anti-desiccant for preventing the stored cells from drying during subsequent cytopathological tests, and a preservative for preventing the stored cells from rotting.

### [Background Art]

The tumor screening system of the present invention operates separation of cells from a vial containing a solution mixed with the cells and collection of the cells on a slide using an automatic unit, and the collection vial for liquid based cytology of the present invention serves to simultaneously store a solution containing cells and pour the solution out of the tumor screening system.

Particularly, the collection vial for liquid based cytology is used to collect cervical cells and mix the collected cells with a solution for testing the cells.

Hereinafter, the collection vials for preserving cells in a solution and testing the cells are referred to as a solution vial.

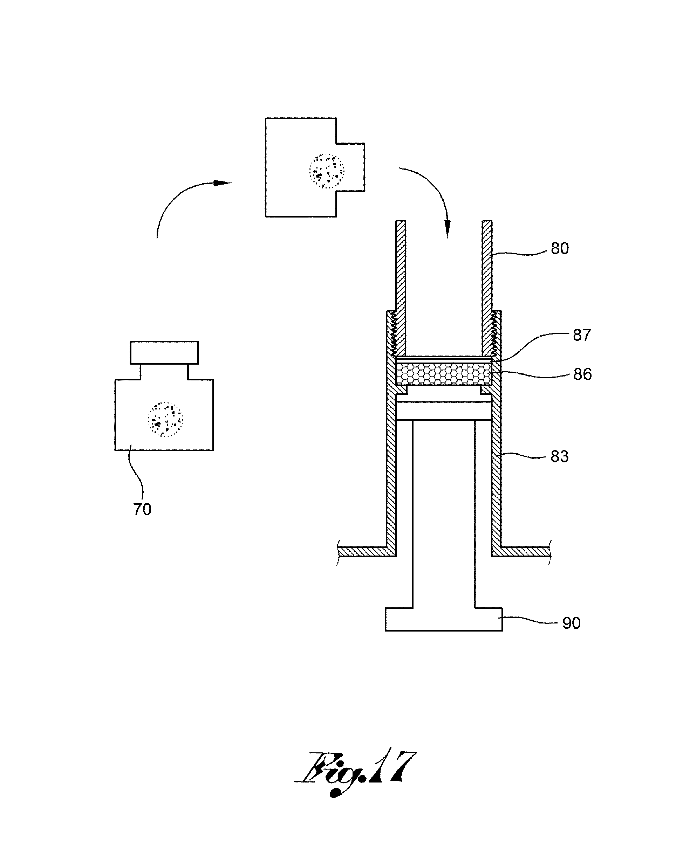

FIG. 17 is a schematic view partially illustrating a conventional solution vial and a conventional tumor screening system. As shown in FIG. 17, the conventional solution vial 70 contains collected cells, and is closed with a lid.

The conventional solution vial 70 is shaken by user s hand or using other devices so that the cells are mixed with a solution in the solution vial 70. The solution vial 70 serves only to store the solution. Accordingly, the conventional tumor screening system comprises a separate container 80 for receiving the solution. That is, the conventional tumor screening system requires the container 80 in addition to the solution vial 70.

Since the container 80 of the conventional tumor screening system has an opened upper end surface, the solution in the container 80 contacts air. A cylindrical main body 83 supports a filter 86 provided with a collection film 87 mounted thereon, and the solution is sucked from the container 80 to the conventional tumor screening system by a piston 90 moving into and out of the main body 83. The main body 83 and the container 80 are connected by screws.

The solution vial for dispersing cells, stuck to a brush, into a solution is installed separately from the conventional tumor screening system. The solution in the solution vial under the condition that the cells are sufficiently dispersed into the solution by shaking the solution vial is poured into the container, a suction device contacting the lower surface of the filter sucks the solution, and then the cells filtered out with the filter are stuck to a slide.

When the container is separated from the main body so as to move the collection film close to the slide, the mixed solution remaining in the container pours out due to gravity, thus not being reused.

Further, since the transfer of the cells from the solution vial to the slide is performed by user's hands, it takes a long time to transfer the cells. Also, contaminants or impurities may be transferred from the user's hands to the slide, thus lowering the accuracy of cytological tests.

After the solution is poured into the container and a desired amount of the solution in the container is sucked by the suction device, the remainder of the solution in the container is already exposed to the air. Accordingly, when the remainder of the solution is reused, it is impossible to assure the accuracy of the tests.

When the filter is separated from the lower portion of the container, the remainder of the solution in the container pours out, and contaminates the system, thus causing sanitary problems.

The brush for liquid based cytology of cervix carcinoma of the present invention facilitates the collection of cells from a woman s uterus.

Most of women's diseases relate to women's genital organs. i.e., uteri.

One of most common women's diseases is cervix carcinoma, which is a kind of tumor. Cervix carcinoma is a tumor having comparatively well-known pathogenesis, and is generated by a Human Papilloma Virus (HPV), which is a kind of venereal disease. In sexual intercourse, when woman's epithelial cells are infected by the HPV, DNA of the HPV penetrates DNA of cell nuclei, propagates, and generates a cancer of the epithelial cells.

Various methods for testing whether or not cells are infected by the HPV, including a cervix cytodiagnosis smear method, have been proposed. Recently, a Polymerase Chain Reaction (PCR) method is used to test whether or not cells are infected by the HPV. The PCR method is the most sensitive and accurate one of screening methods for early diagnosis of cervix carcinoma.

In order to use the PCR method, the collection of cells of a woman's uterus, particularly cells of a women's cervix, is required. Brushes haying various shapes for collecting cells from a woman's uterus have been proposed.

Korean Utility Model Laid-open Publication No. 1999-1000001 (hereinafter, referred to as "reference 1") discloses 'cell collecting structure for diagnosing cervix disease'. The reference 1 discloses 'brush for collecting cervical cells', which is inserted into a woman's vagina and collects the cervical cells.

The 'cell collecting structure for diagnosing cervix disease' disclosed in reference 1 comprises an external membrane collecting unit and an internal membrane collecting unit detachably, which are connected to both sides of a handle, thereby causing inconvenience of separately collecting cells from the external membrane and the internal membrane of the cervix.

Further, Korean Patent Laid-open Publication No. 2004-82781 (hereinafter, referred to as "reference 2") discloses 'apparatus for collecting cervical cells'. The 'apparatus for collecting cervical cells' disclosed in reference 2 comprises a main collector made of silicon, a subsidiary collector, and a joint having an adjustable length.

The 'apparatus for collecting cervical cells' disclosed in reference 2 is advantageous in that the length of the apparatus is adjustable, but is disadvantageous in that the collection of the cells depends only on a collecting brush prepared at a first collecting unit and thus a cell collecting efficiency is not assured. Further, a second collecting unit is extended from the first collecting unit in a direction perpendicularly to the lengthwise direction of the first collecting unit, and stimulates the inner wall of a woman's vagina when the 'apparatus for collecting cervical cells' is inserted into the woman's vagina, thereby causing pain to a patient or an injury to the inner wall of the vagina.

In order to use the PCR method, collected cells are generally stored in a container filled with a cell fixing solution. In the structure and apparatus disclosed in references 1 and 2, since collecting units are directly separated from the brushes by operator's hands or using other tools, such as clamps, the collecting units may be contaminated with foreign substances. This obstructs the accurate diagnosis of the collected cells.

The supporting solution for cytodiagnosis of the present invention comprises a buffer agent for storing collected cells under the condition that the collected cells are maintained in a designated pH range, alcohol for suppressing the protein degeneration of the cells to maintain the pathological structures of the cells, an anti-desiccant for preventing the stored cells from drying during subsequent cytopathological tests, and a preservative for preventing the stored cells from rotting.

The supporting solution for cytodiagnosis refers to a solution for preserving cells collected from a human body to perform liquid based cytology of the cells for cytopathological diagnosis. The liquid based cytology is used to test gynecological samples, such as cervical cells, and non-gynecological samples, such as sputum, body fluids, and urine, and to perform fine needle aspiration biopsy. In the liquid based cytology, whether or not cancer cells exist in collected cells is checked by inspecting the collected cells using a microscope.

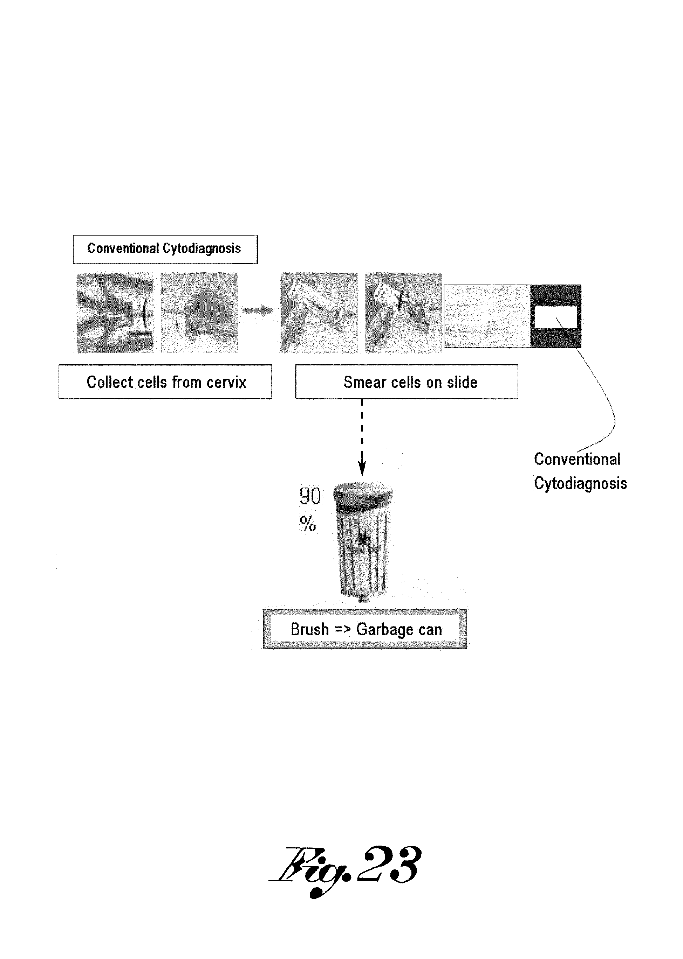

FIG. 23 is a schematic view illustrating conventional cytodiagnosis. With reference to FIG. 23, the conventional cytodiagnosis applied a pap smear method, in which cells are smeared on a slide and are then tested. When the cells are smeared on the slide, the cells on the slide are easily dried and a viscous solution and erythrocyte are not removed from the cells, thereby causing a difficulty in reading diagnosis results. Further, since only 10% of cells collected from a woman's cervix using a brush are smeared on the slide and the remainder of the collected cells are thrown away, cells, which are important to the diagnosis, may not be smeared on the slide. Accordingly, the conventional cytodiagnosis has a low reliability.

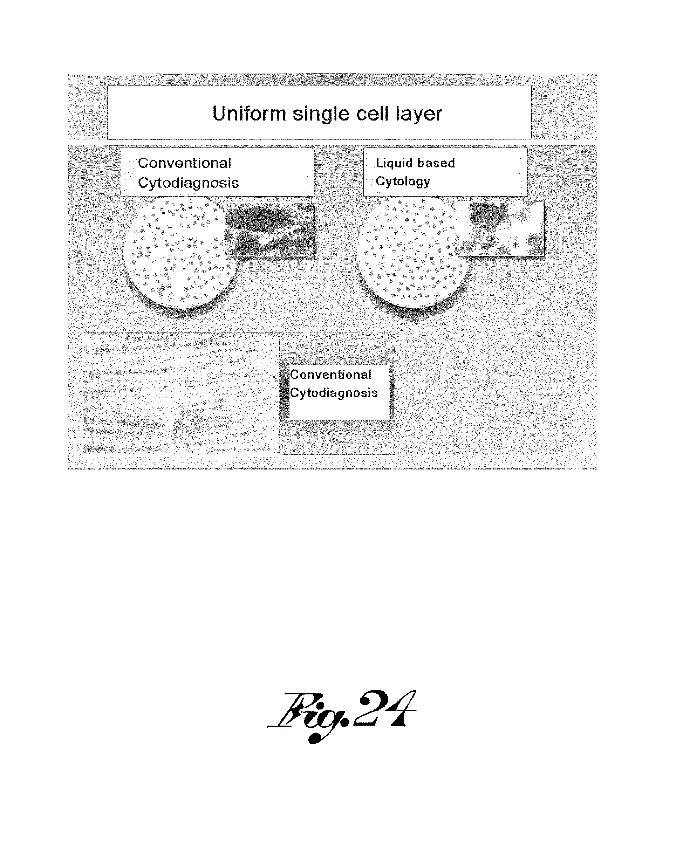

In order to solve the above problem, liquid based cytology, in which collected cells are preserved in a supporting solution so as to perform cytopathological diagnosis of the cells, has been developed now. In the liquid based cytology, cells are collected using a brush and are preserved in a supporting solution, and when necessary whether or not the cells preserved in the supporting solutions are abnormal is judged by tests. FIG. 24 is a view for comparing accuracies of the conventional cytodiagnosis and the liquid based cytology. With reference to FIG. 24, in accordance with the conventional cytodiagnosis, cells of a sample are not uniformly distributed on a slide, and thus diagnosis results change according to portions of the sample to be tested. On the other hand, in accordance with the liquid based cytology, cells of a sample are uniformly distributed on a slide, and thus accuracy in diagnosis is increased.

In order to perform the above liquid based cytology, a solution for preserving collected cells is required. Conventionally, the liquid based cytology requires 95% ethanol. The 95% ethanol cannot preserve cells for a long period of time (can preserve cells for approximately 1 week), and cannot remove erythrocyte from the cells, thus requiring a separate process for removing the erythrocyte from the cells. Further, nuclei of the cells preserved in the 95% ethanol are deformed and thus it is impossible to extract DNA from the cells. Moreover, the 95% ethanol has a high alcohol content and is inflammable, thus causing a danger of fire.

In order to solve the above problems, U. S. Patent Serial No. 5,256,571 discloses a cell preservative solution comprising a water-miscible alcohol, an anti-clumping agent, and a buffer agent. In the cell preservative solution, the alcohol is one selected from the group consisting of ethanol, isopropanol, and methanol, the anti-clumping agent is one selected from the group consisting of ethylenediamine tetraacetic acid and salts thereof, and the buffer agent is one selected from the group consisting of ethylenediamine tetraacetic acid, ethylenediamine tetraacetic acid salts, citric acid, and citric acid salts. However, since the amount of the alcohol for fixing the cells is 45-55 parts by weight, preferably 50 parts by weight, the cell preservative solution disclosed in U. S. Patent Serial No. 5,256,571 is still inflammable. Further, the cell preservative solution has a low erythrocyte removal rate, preserves the cells: only for a comparatively short period of time (approximately 3 months), and causes a difficulty in extracting DNA from the cells.

### [Disclosure]

### [Technical Problem]

Therefore, the present invention has been made in view of the above problems, and it is an object of the present invention to provide a tumor screening system, which operates separation of cells from a vial containing a solution mixed with the cells, and collection of the cells on a slide using an automatic unit.

It is a further object of the present invention to provide a collection vial for liquid based cytology serving to simultaneously store a solution containing cells and pour the solution out of the tumor screening system.

It is another object of the present invention to provide a brush for liquid based cytology of cervix carcinoma, which uniformly collects cells from various regions of a woman's cervix, such as an inner wall and a transvaginal portion of cervix, without damage to the inner wall of a woman's vagina.

It is yet another object of the present invention to provide a supporting solution for cytodiagnosis, which preserves collected cells for a long period of time while maintaining morphological structures of the collected cells, removes erythrocyte from the preserved cells so that the cells are easily and accurately tested, allows DNA to be extracted from the preserved cells, prevents the preserved cells from drying during tests, is nonflammable, and thus is easy to handle.

### [Technical Solution]

In accordance with an aspect of the present invention, the above and other objects can be accomplished by the provision of a tumor screening system comprising solution vial operating units, each comprising a fixing unit for fixing a solution vial having a piston installed therein, and a cylinder device for linearly moving the solution vial toward a filter connected to the solution vial; piston operating units, each comprising a cylinder fixed to the corresponding solution vial operating unit, and a cylinder rod installed on the cylinder and fixed to one portion of the piston of the solution vial, for linearly moving piston; and suction units, each comprising a connecting portion disposed under the corresponding solution vial operating unit and connected to the filter, and a tube connected to the connecting portion for guiding a solution filling the solution vial, and a suction device connected to the inner space of the tube.

Preferably, the tumor screening system may further comprise slide operating units, each comprising a slide mounting portion on which a slide is mounted, and a moving device for moving the slide mounting portion to achieve contact between the slide and the filter connected to the corresponding suction unit.

Preferably, the tumor screening system may further comprise alcohol receiving units for receiving alcohol, and each of the slide operating units may further comprise a slide dropping unit for dropping the slide into the corresponding alcohol receiving unit.

Preferably, the tumor screening system may further comprise filter operating units, each comprising a moving unit for moving the filter to a position under the solution vial, and a fixing unit for fixing the filter.

Preferably, the tumor screening system may further comprise mixed solution forming units, each comprising a connecting portion, to which the solution vial is connected, and a rotating portion for rotating the connecting portion.

Preferably, the tumor screening system may further comprise lid open units, each comprising a moving portion approaching a position between the corresponding solution vial operating unit and the corresponding suction unit, and a rotating device installed on the moving portion, connected to a lid installed on the corresponding solution vial, and rotated.

The piston in the solution vial may comprise a piston rod having grooves formed therein and snap-connected to protrusions formed on two opposite flexible sheets of the solution vial so that the piston is fixed to a designated position of the solution vial, and the tumor screening system may further comprise piston connection releasing units, each comprising tapered protrusions, so that the two flexible sheets are inserted into a space between the protrusions, and fixed to one side of the system, a cylinder installed above the protrusions so that the cylinder is separated from the protrusions by a designated interval, and a cylinder rod inserted into and taken out of the cylinder.

In accordance with a further aspect of the present invention, there is provided a solution vial comprising a casing comprising a piston guide slit formed in one end of an inner surface thereof forming an inner space and an opening formed through the other end of the inner surface thereof; a gauze film installation unit connected to the casing, and comprising a through hole and a gauze film installed at a designated position of the through hole; a piston comprising a contact portion contacting the inner surface of the casing, and a rod portion extended from the contact portion in one direction and inserted into the piston guide slit; and a lid detachably connected to the gauze film installation unit for opening and closing the gauze film installation unit in one direction.

Preferably, impellers may be protruded from the contact portion of the piston towards the inner space of the casing.

Preferably, agitating protrusions for agitating a solution received in the solution vial may be formed on the inner surface of the casing in a circumferential direction.

Preferably, the piston guide slit may comprise a pair of planes elastically deformed and piston supporting protrusions formed on the planes in directions, which face each other; and grooves, into which the piston supporting protrusions are inserted, may be formed in the rod portion of the piston.

Preferably, a filter connecting flange connected to a flange surrounding a collecting film of a filter may be formed on the gauze installation unit.

Preferably, the gauze film installation unit may be connected to the casing by screws, and holding protrusions may be formed on the outer circumferential surface of the gauze film installation unit.

In accordance with another aspect of the present invention, there is provided a brush for liquid based cytology used to collect cells of a woman's cervix, comprising a holding unit comprising an insertion portion having a protrusion formed on one end thereof and forming an inner sliding space therein; a cell collecting member comprising a first collecting unit comprising a plurality of collecting protrusions, a first hook-latched portion, and second hook-latched portions; a second collecting unit extended from the first collecting unit in the lengthwise direction of the holding unit and elastically expanding and contracting in the lengthwise direction of the holding unit; and a first connecting unit formed integrally with one end of the second collecting unit close to the holding unit; an inner detachable member comprising a second connecting unit latched onto and released from the protrusion of the holding unit so as to be detachably connected to the holding unit; a first hook latched onto the first hook-latched portion of the first collecting unit; and a connecting member for connecting the second connecting unit and the first hook; and a movable member comprising second hooks formed at one end thereof close to the cell collecting member and latched onto and released from the second hook-latched portions, and installed in the holding unit so that the movable member slides from a first latched position, at which the second hooks are latched onto the second hook-latched portions, to a second latched position, at which the first hook is latched onto the first hook-latched portion according to the approach of the first collecting unit to the first connecting unit due to the contraction of the second collecting unit under the condition that the second hooks are latched onto the second hook-latched portions.

The second connecting unit of the inner detachable member may have an approximately cylindrical structure having a through hole so that the movable member passes the through hole in the lengthwise direction of the holding unit; and the connecting member of the inner detachable member may have an approximately V beam structure, both ends of which are connected to the second connecting unit and the first hook is formed on a valley portion of the V beam-structured connecting member towards the first hook-latched portion.

The second collecting unit may comprise a plurality of collecting combs, which are separated from each other by a designated interval along the circumferential direction centering on a lengthwise axis of the holding unit, and connect the first collecting unit and the first connecting unit; and the collecting combs may be elastically bent and contract radially towards the outside of the lengthwise axis of the holding unit so as to cause the first collecting unit to approach the first connecting unit by the sliding of the movable member from the first latched position to the second latched position.

An insertion hole, into which the second connecting unit of the inner detachable member is inserted at the first and second latched positions of the movable member, may be formed through the first connecting unit of the cell collecting member.

A first guide rib may be protruded from one of the inner wall of the insertion hole of the first connecting unit and the outer wall of the second connecting unit in the lengthwise direction of the holding unit; and a first guide groove for guiding the first guide rib when the second connecting unit is inserted into the insertion hole of the first connecting unit may be formed in the other one of the inner wall of the insertion hole of the first connecting unit and the outer wall of the second connecting unit.

Extended branches creating an approximately V beam shape may be formed at the end of the movable member close to the cell collecting member; and the second hooks may be respectively protruded from both ends of the extended branches towards the opposite ends.

A second guide rib may be formed on the inner wall of the second connecting unit of the inner detachable member in the lengthwise direction of the holding unit; and a second guide groove for guiding the sliding of the second guide rib when the insertion portion of the holding unit is inserted into the second connecting unit of the inner detachable member, and third guide grooves for guiding the sliding of the extended branches of the movable member in the lengthwise direction of the holding unit may be formed in the insertion portion of the holding unit.

At the second latched position of the movable member, the second hooks of the movable member may be released from the second hook-latched portions of the cell collecting member, and pass through the through hole of the second connecting unit of the inner detachable member from the end thereof close to the cell collecting member to the end thereof close to the holding unit, thereby allowing the movable member to slidably move to a latch-releasing position in the sliding space of the holding unit.

Pressing portions, for pressing the end of the second connecting unit of the inner detachable member close to the holding unit when movable member moves from the latch-releasing position to the cell collecting member, may be formed on the ends of the extended branches of the movable member; and when the pressing portions press the first connecting unit at a force having at least a designated intensity, the protrusion of the holding unit may be released from the second connecting unit of the inner detachable member, thereby separating the inner detachable member, which is connected to the cell collecting member, from the holding unit.

Inclined planes contacting the inner walls of the third guide grooves for allowing the extended branches to approach each other may be formed on at least portions of side surfaces of the extended branches so that the extended branches approach each other and the ends of the extended branches easily pass through the through hole of the inner detachable member when the movable member slides from the second latched position to the latch-releasing position.

The inner detachable member may further comprise a baffle flange extended outwardly from the second connecting unit for preventing the first connecting unit from retreating to the holding unit due to the elastic force of the second collecting unit at the second latched position.

In accordance with yet another aspect of the present invention, there is provided a supporting solution for cytodiagnosis comprising a buffer agent for maintaining the acidity of the supporting solution in a designated pH range; alcohol; and an anti-desiccant for preventing the cells from drying during subsequent tests.

Preferably, the buffer agent may maintain the acidity of the supporting solution to be pH 2 to 6.5.

Preferably, the buffer agent may be one selected from the group consisting of lactic acid, citric acid, and phosphoric acid.

Preferably, the buffer agent may have an amount of 10 to 40 parts by weight.

Preferably, the alcohol may be one selected from the group consisting of ethanol, propanol, isopropyl alcohol, butanol, and pentanol.

Preferably, the alcohol may be one selected from the group consisting of ethanol, propanol, and isopropyl alcohol.

Preferably, the alcohol may have an amount of 5 to 40 parts by weight.

More preferably, the alcohol may have an amount of 10 to 35 parts by weight.

Preferably, the anti-desiccant may be polyhydric alcohol.

Preferably, the polyhydric alcohol may be one selected from the group consisting of ethylene glycol, propylene glycol, and glycerol.

Preferably, the anti-desiccant may be polyethylene glycol or polypropylene glycol.

Preferably, polyethylene glycol or polypropylene glycol may have a molecular weight of 100 to 100,000Da.

Preferably, the anti-desiccant may have an amount of 0.1 to 30 parts by weight.

Preferably, the anti-desiccant may have an amount of 0.1 to 15 parts by weight.

Preferably, the supporting solution may further comprise a preservative.

Preferably, the preservative may comprise at least one selected from the group consisting of ascorbic acid, sorbic acid, citric acid, salicylic acid, sodium benzoate, formalin, glyoxal, and calcium propionate.

Preferably, the preservative may have an amount of 0.1 to 20 parts by weight.

### [Advantageous Effects]

The tumor screening system of the present invention operates separation of cells from a vial containing a solution mixed with the cells and collection of the cells on a slide using an automatic unit.

The collection vial for liquid based cytology of the present invention serves to simultaneously store a solution containing cells and pour the solution out of the tumor screening system.

The brush for liquid based cytology of cervix carcinoma of the present invention uniformly collects cells from various regions of a woman's cervix, such as an inner wall and a transvaginal portion of cervix, without damage to the inner wall of a woman's vagina. Further, the brush allows the collected cells to be safely stored in a designated vial, thereby assuring stable and accurate cell collection and diagnosis.

The supporting solution for cytodiagnosis of the present invention has several effects, as follows.

First, the supporting solution preserves collected cells for a long period of time while maintaining morphological structures of the collected cells.

Second, the supporting solution removes erythrocyte from the preserved cells so that the cells are easily and accurately tested.

Third, the supporting solution is nonflammable, thus being easy to handle.

Fourth, the supporting solution is excellently stained, thus being easy to accurately diagnose.

Fifth, the supporting solution uses the remainder of the preserved cells without recollecting cells from a patient in gene analysis.

### [Description of Drawings]

The above and other objects, features and other advantages of the present invention will be more clearly understood from the following detailed description taken in conjunction with the accompanying drawings, in which:
FIG. 1 is an exploded perspective view of a solution vial in accordance with a preferred embodiment of the present invention;
FIG. 2 is a perspective view of the solution vial in accordance with the preferred embodiment of the present invention;
FIG. 3 is a partially exploded perspective view of the solution vial in accordance with the preferred embodiment of the present invention, for illustrating the snap-connection of a casing and a piston;
FIG. 4 is a partially exploded perspective view of the solution vial in accordance with the preferred embodiment of the present invention;
FIG. 5 is a perspective view of an upper portion of a filter used in a tumor screening system in accordance with the present invention;
FIG. 6 is a schematic perspective view illustrating the structure of the filter used in the tumor screening system in accordance with the present invention;
FIG. 7 is a perspective view of a lower portion of the filter used in the tumor screening system in accordance with the present invention;
FIG. 8 is a perspective view of a two row-type tumor screening system in accordance with the present invention;
FIG. 9 is a schematic side view of a mixed solution forming unit of the tumor screening system in accordance with the present invention;
FIG. 10 is a partially enlarged view of the tumor screening system of FIG. 9;
FIG. 11 is a partial sectional view illustrating the connection of a piston of a solution vial and an end of a cylinder rod of a piston operating unit of the tumor screening system in accordance with the present invention;
FIG. 12 is a schematic longitudinal sectional view of a lid open unit of the tumor screening system in accordance with the present invention;
FIG. 13 is a partial side view of a slide operating unit of the tumor screening system in accordance with the present invention;
FIG. 14 is a schematic sectional view of a suction unit of the tumor screaming system in accordance with the present invention:
FIG. 15 is a schematic front view illustrating the connection of the solution vial to a piston connection releasing unit of the tumor screening system in accordance with the present invention;
FIG. 16 is a schematic side view illustrating the connection of the solution vial to the piston connection releasing unit of the tumor screening system in accordance with the present invention;
FIG. 17 is a schematic view partially illustrating a conventional solution vial and a conventional tumor screening system;
FIG. 18 is a perspective view of a brush for liquid based cytology in accordance with the present invention;
FIG. 19 is an exploded perspective view of the brush for liquid based cytology of FIG. 18;
FIG. 20 is a sectional view taken along the line III-III of FIG. 18;
FIGS. 21 and 22 are sectional views for illustrating operating states of the brush for solution base cytology in accordance with the present invention;
FIG. 23 is a schematic view illustrating conventional cytodiagnosis;
FIG. 24 is a view for comparing accuracies of the conventional cytodiagnosis and solution base cytology;
FIG. 25 is a view illustrating collected cells in an initial state, which start to be preserved in a supporting solution for cytodiagnosis of one example of the present invention;
FIG. 26 is a view illustrating cells in a state, which have been preserved for at least 18 months in the supporting solution for cytodiagnosis of one example of the present invention;
FIG. 27 is a microphotograph of cells preserved in a solution of one comparative example;
FIG. 28 is a microphotograph of cells preserved in a supporting solution for cytodiagnosis of one example of the present invention;
FIGS. 29 and 30 are photographs illustrating staining of cells preserved in supporting solutions for cytodiagnosis of examples of the present invention;
FIG. 31 is a photograph illustrating staining of cells preserved in a solution of one comparative example;
FIGS. 32 and 33 are electrophoretic photographs of cells preserved in supporting solutions for cytodiagnosis of examples of the present invention; and
FIG. 34 is an electrophoretic photograph of cells preserved at room temperature in the supporting solution for cytodiagnosis of one example of the present invention and in a solution of one comparative example.

### [Best Mode]

Hereinafter, a solution vial (a collection vial for liquid based cytology and test) in accordance with a preferred embodiment of the present invention will be described in detail with reference to the annexed drawings.

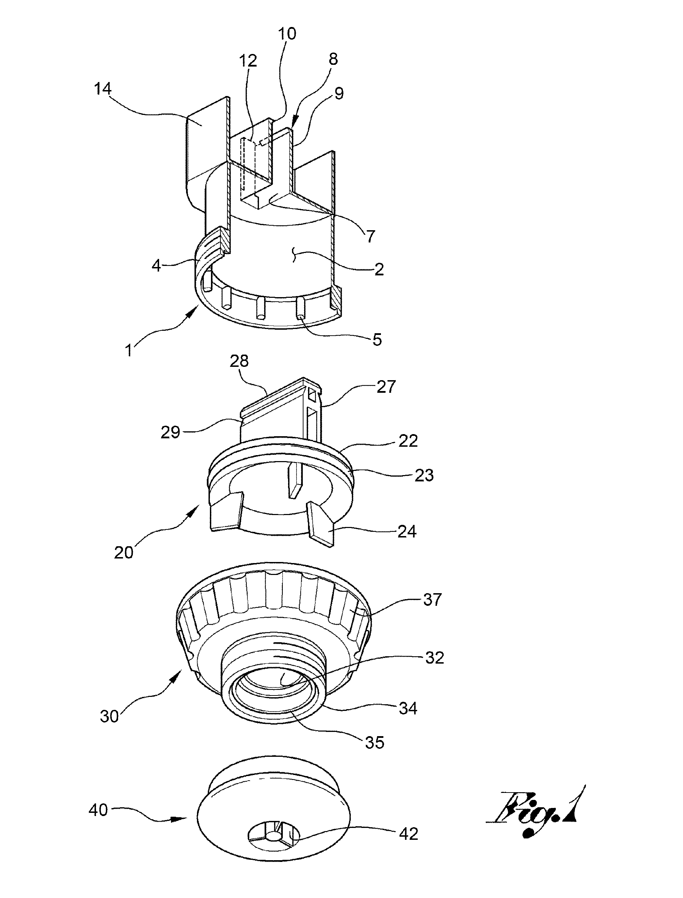

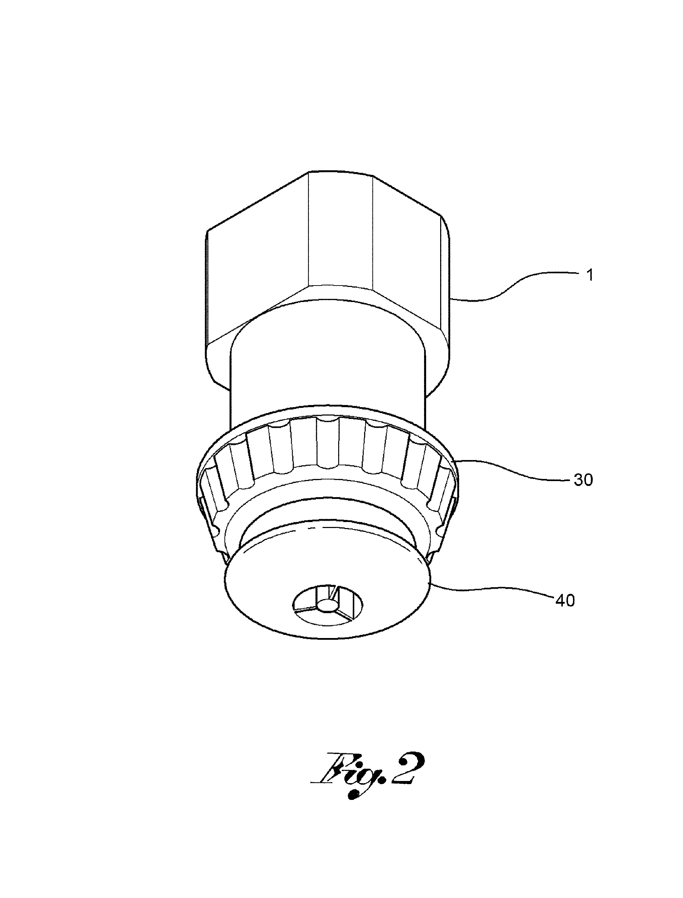

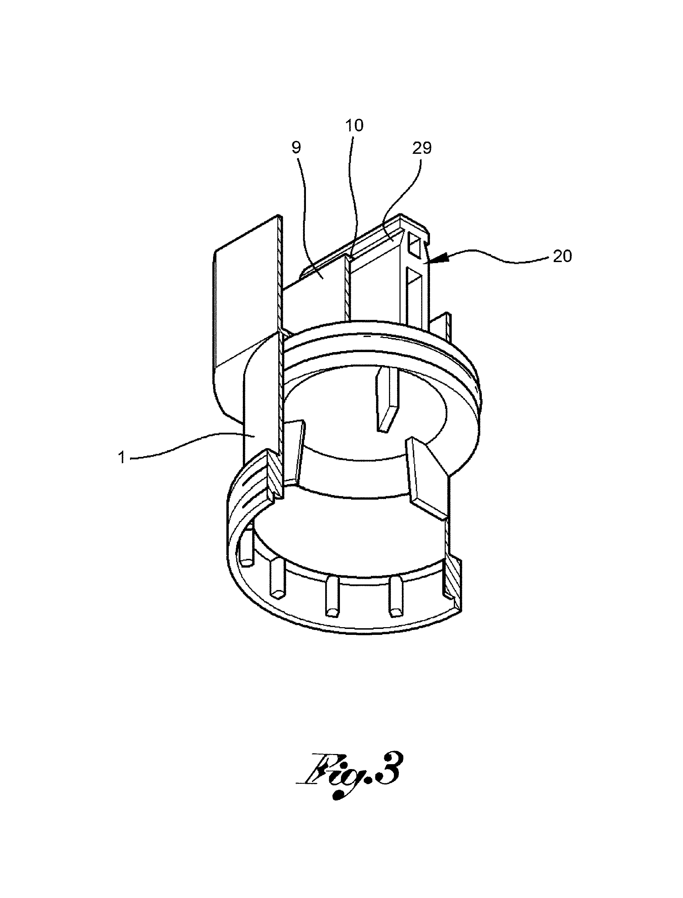

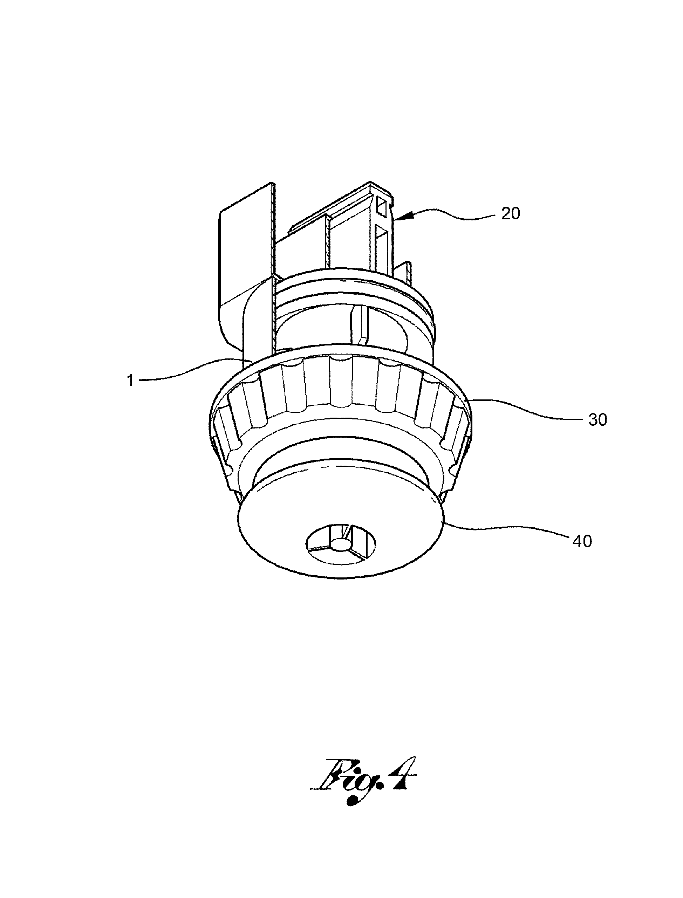

FIG. 1 is an exploded perspective view of a solution vial in accordance with the preferred embodiment of the present invention, FIG. 2 is a perspective view of the solution vial in accordance with the preferred embodiment of the present invention, FIG. 3 is a partially exploded perspective view of the solution vial in accordance with the preferred embodiment of the present invention, for illustrating the snap-connection of a casing and a piston, and FIG. 4 is a partially exploded perspective view of the solution vial in accordance with the preferred embodiment of the present invention.

As shown in FIGS. 1 to 4, the solution vial of the present invention comprises a casing 1 having an inner space for receiving a solution, a piston 20 installed in the casing 1, a gauze film installation unit 30 connected to one end of the casing 1, and a lid 40 connected to the gauze film installation unit 30. The casing 1, the piston 20, the gauze film installation unit 30, and the lid 40 are made of synthetic resins.

A solution receiving portion 2 having a smooth inner surface for forming a cylindrical inner space is located at the center of the casing 1, and a solution agitating portion 4, which has an inner diameter larger than that of the solution receiving portion 2 and opened ends, and is provided with agitating protrusions 5 formed on the inner surface thereof and a screw portion connected to the gauze film installation unit 30 by screws, is located under the solution receiving portion 2. The upper end surface of the solution receiving portion 2 is closed except for a rectangular through hole 7, and a guide slit 8, which has a rectangular channel structure and is connected to the through hole 7, is formed through the side of the casing 1 opposite to the solution receiving portion 2. The guide slit 8 includes a pair of first planes 9, which have a relatively broad width and are made of an elastic material, a pair of second planes 12, which are respectively perpendicular to the corresponding first planes 9 and are made of an elastic material, and fixing protrusions 10, which have a trigonal prism structure and are respectively formed on ends of the first planes 9. Flat planes 14, which are symmetric with each other, are formed on the upper portion of the outer surface of the casing 1. The outer diameter of the portion of the casing 1 under the flat planes 14 is decreased, and the outer diameter of the solution agitating portion 4 is increased again.

The piston 20 is installed in the solution receiving portion 2 of the casing 1, and comprises a cylindrical contact portion 22 having a side surface contacting the inner surface of the solution receiving portion 2, and a rod portion 27 having a rectangular cross section and perpendicularly extended from the contact portion 22 in one direction. A groove is formed in the central portion of the side surface of the contact portion 22, and a sealing rubber ring 23 is inserted into the groove. A plurality of impellers 24 are protruded downwardly from the lower surface of the contact portion 22. The rod portion 27 is installed in the casing 1 via the rectangular through hole 7 formed through the upper end surface of the solution receiving portion 2, an upper end 28 of the rod portion 27 is tapered so that the tapered upper end 28 can push the first planes 9 of the guide slit 8 out and pass between the fixing protrusions 10, and connection grooves 29 are formed in the rod portion 27 under the tapered upper end 28 so that the connection grooves 29 are snap-connected to the fixing protrusions 10 of the guide slit 8. The upper portion of the rod portion 27 is tapered, and a through hole is formed through the tapered upper portion of the rod portion 27 in the lengthwise direction.

The gauze film installation unit 30 has a hollow structure so that a screw portion formed in the inner surface of the gauze film installation unit 30 is connected to the solution agitating portion 4 of the casing 1, and a gauze film 32 having pores for preventing the solution from rapidly passing through the solution vial is installed at the center of the gauze film installation unit 30. A cylindrical outer wall 34 and a cylindrical inner connection wall 35 separated from the cylindrical outer wall 34 by a designated interval are formed on the lower portion of the gauze film installation unit 30. A screw portion connected to the lid 40 is formed on the outer surface of the outer wall 34, and the inside of the inner connection wall 35 forms a channel connected to the gauze film 32. Protrusions 37 are formed on the outer surface of the gauze film installation unit 30, thereby allowing a user to conveniently screw-connect the gauze film installation unit 30 to the casing 1 and to easily release the connection.

The lid 40 is connected to the gauze film installation unit 30. A screw portion connected to the outer wall 34 of the gauze film installation unit 30 is formed on one surface of the lid 40, and three connector insertion holes 42 are formed in the other surface of the lid 40.

Thereafter, a filter 50 used together with the solution vial of the present invention will be descried with reference to the annexed drawings.

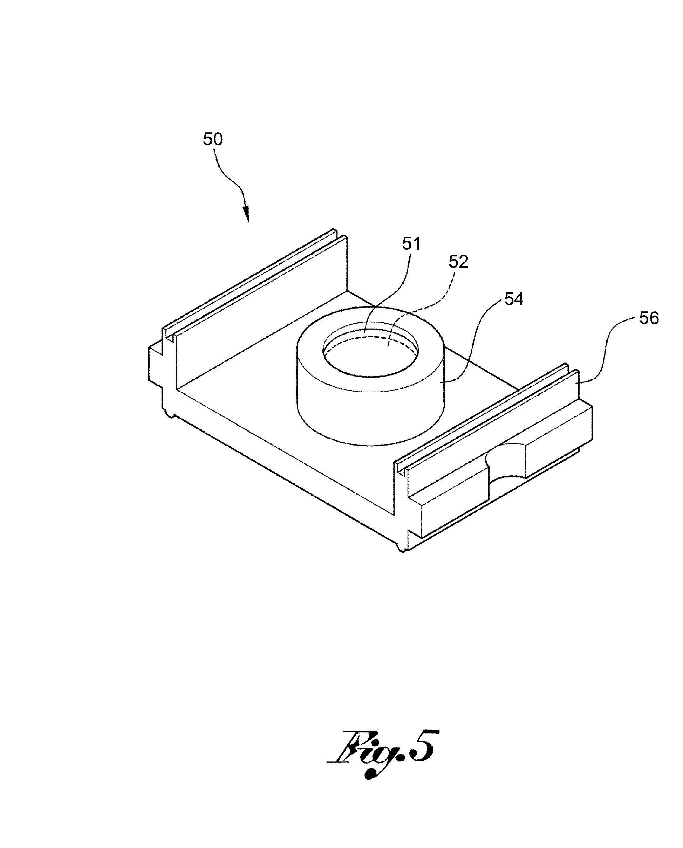

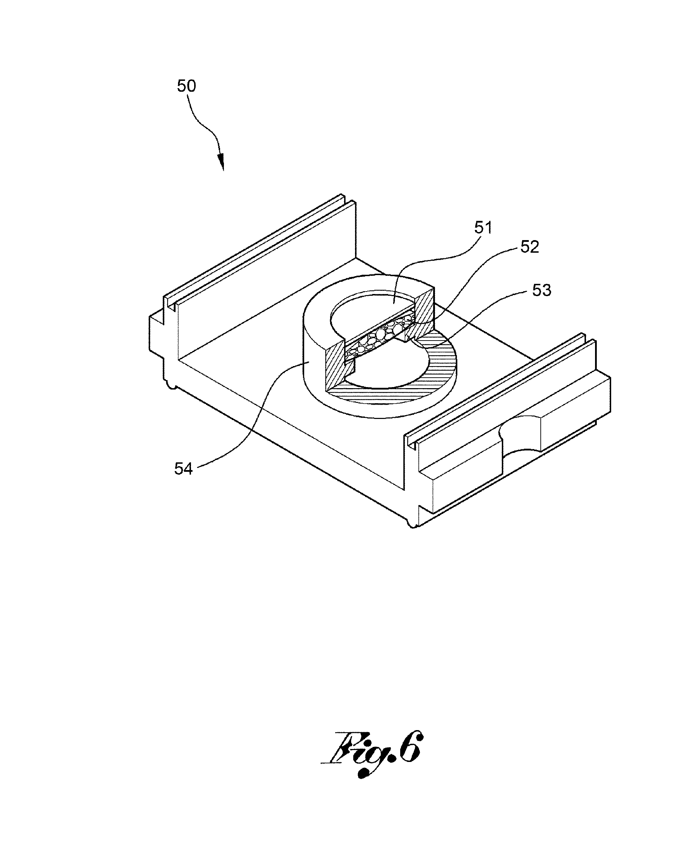

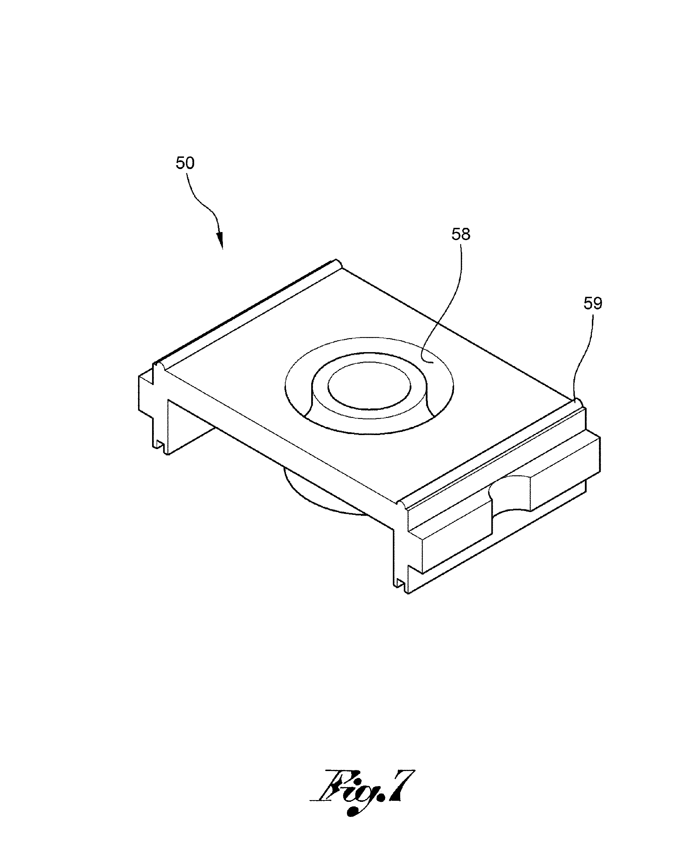

FIG. 5 is a perspective view of an upper portion of a filter used in a tumor screening system in accordance with the present invention, FIG. 6 is a schematic perspective view illustrating the structure of the filter used in the tumor screening system in accordance with the present invention, and FIG. 7 is a perspective view of a lower portion of the filter used in the tumor screening system in accordance with the present invention.

As shown in FIGS. 5 to 7, the filter 50 comprises a supporter 53 for supporting a cylindrical member 52, which has a coin-shaped cross section and air/water permeability and supports a collection film 51 having a laminated structure and provided with pores formed therethrough, a cylindrical connection flange 54 installed on the upper surface of the filter 50 around the supporter 53 and inserted into a space between the outer wall 34 and the inner connection wall 35 of the gauze film installation unit 30, upper guide members 56 having a designated height rectilinearly protruded from the upper surface of the filter 50 at both sides of the connection flange 54 so that a plurality of filters 50 can be vertically stacked and the stacked filters 50 slide against each other, and rectilinear grooves respectively formed in upper ends of the upper guide members 56 in the lengthwise direction of the upper guide members 56. A cylindrical connection groove 29 is formed in the center of the lower surface of the filter 50 so that a cylindrical protruding flange of the tumor screening system can be inserted into the cylindrical connection groove 29, and lower guide members 59 inserted into the rectilinear grooves of the upper guide members 56 of another filter 50 are formed at both sides of the cylindrical connection groove 29.

Thereafter, a tumor screening system using the solution vial of the present invention will be descried with reference to the annexed drawings.

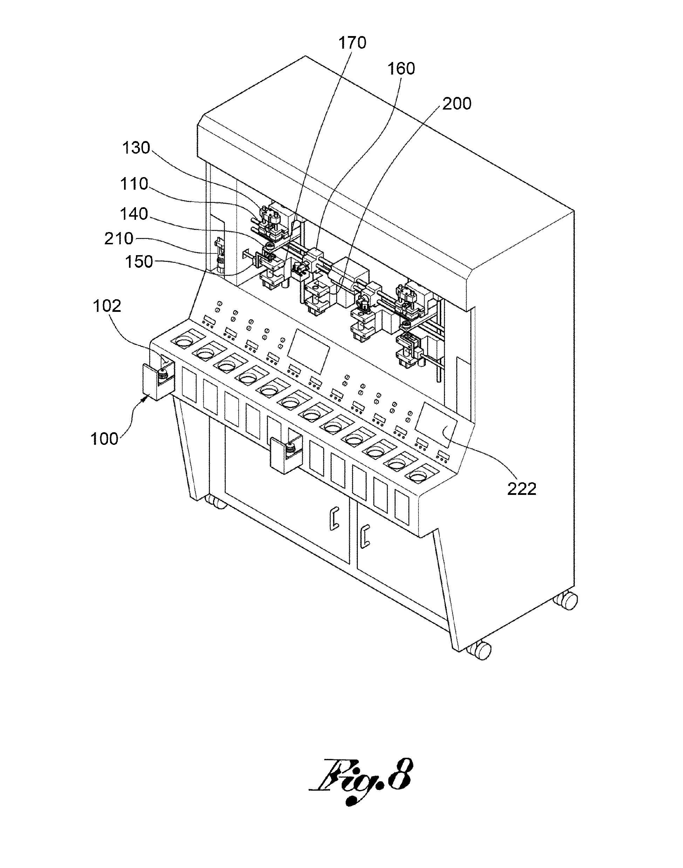

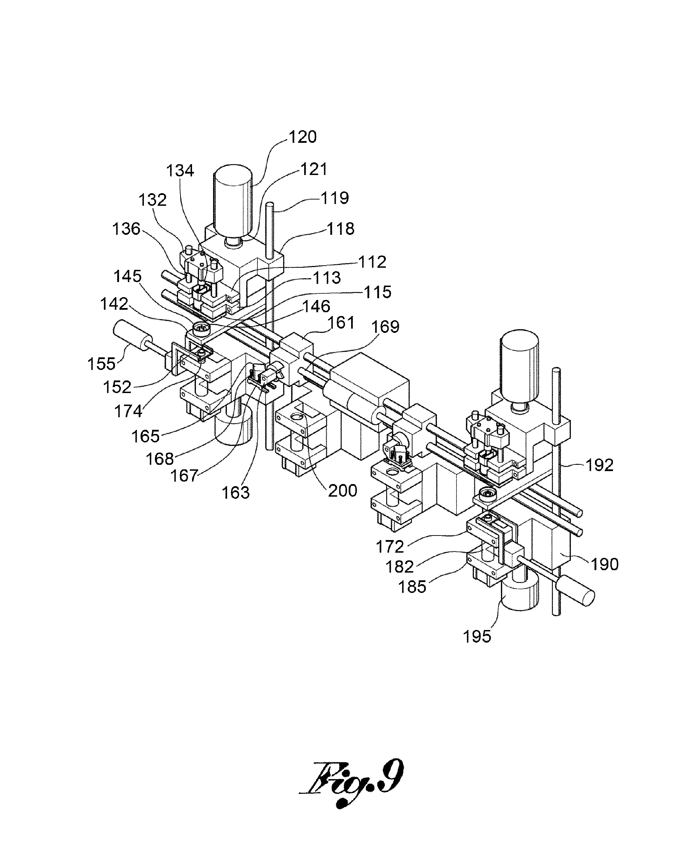

FIG. 8 is a perspective view of a two row-type tumor screening system in accordance with the present invention, and FIG. 9 is a partially enlarged view of the tumor screening system of FIG. 8. As shown in FIGS. 8 and 9, the tumor screening system comprises mixed solution forming units 100 for uniformly mixing cells with a solution filling the solution vials by rotating solution vials, solution vial operating units 110 for operating the solution vial, piston operating units 130 for operating the piston 20 installed in the solution vial, lid open units 140 for automatically opening and closing the lid 40 of the solution vial, filter operating units 150 for operating the filter 50, slide operating units 160 for operating a slide so that the slide contacts the filter 50, suction units 170 for sucking the mixed solution from the solution vial, alcohol receiving units 200 for receiving alcohol in which the slide is soaked, and piston connection releasing units 210 for separating the piston 20 from the solution vial.

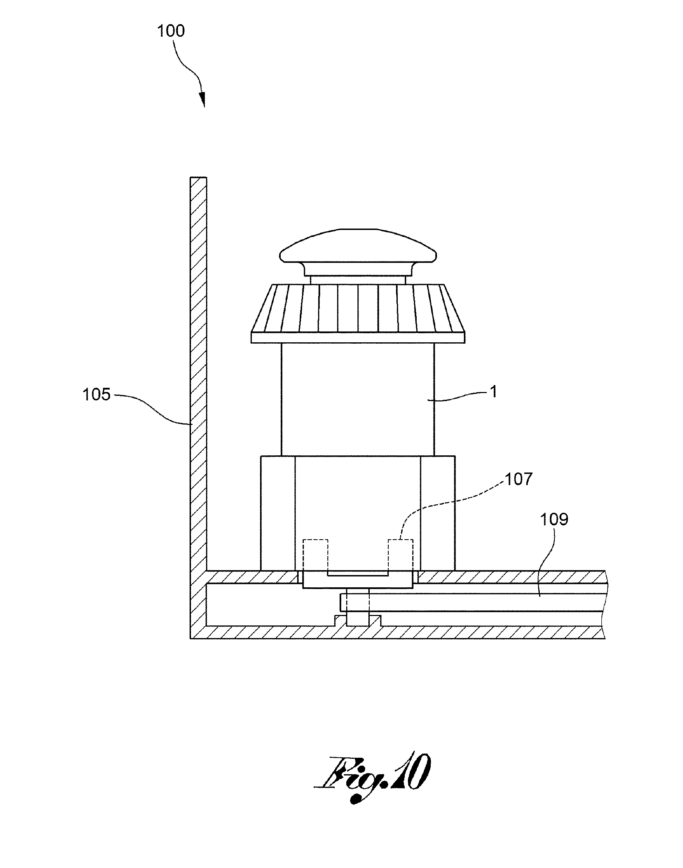

FIG. 10 is a schematic side view of the mixed solution forming unit of the tumor screening system in accordance with the present invention.

As shown in FIGS. 8 and 10, each of the mixed solution forming units 100 comprises a drawer-type receiving portion 105 inserted into and taken out of an agitating chamber 102 and having a space for receiving the solution vial, and a rotator 107 having protrusions connected to the casing 1 of the solution vial and connected to a motor (not shown) by a chain 109. The mixing of cells with the solution of the solution vial by the mixed solution forming unit 100 is achieved by rotating the solution vial using the rotator 107, after the receiving portion 105 is taken out of the agitating chamber 102, the solution vial is connected to the rotator 107, and then the receiving portion 105 is returned to the inside of the agitating chamber 102.

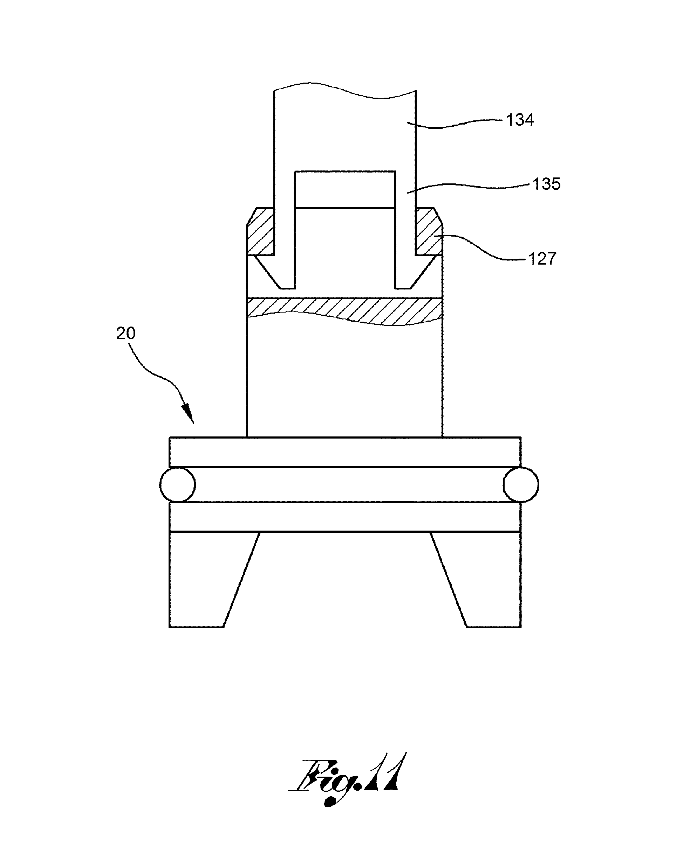

As shown in FIGS. 8 and 9, each of the solution vial operating units 110 comprises two-step blocks 112 and 113 vertically arranged in parallel for respectively supporting portions of the casing 1 of the solution vial having different outer diameters, the lowermost block 115 fixed to the lower block 113 of the two-step blocks 112 and 113 by a spring (not shown), and a moving block 118, to which the two-step blocks 112 and 113 are fixed, moving along a linear guide 119 for moving the two-step blocks 112 and 113. One side of the moving block 118 is fixed to a cylinder rod 121 of a fixed pneumatic cylinder 120. The lowermost block 115 has a hollow structure. The inner diameter of the lowermost block 115 is smaller than the outer diameter of the gauze film installation unit 30 so that the lowermost block 115 catches the gauze film installation unit 30, so as not to release the solution vial from the solution vial operating unit 110, and restricts the rotation of the gauze film installation unit 30. Each of the piston operating units 130 comprises a pneumatic cylinder 132 disposed above the two-step blocks 112 and 113 of each of the solution vial operating units 110 and fixed to the two-step blocks 112 and 113 through connection rods 136, and a cylinder rod 134 connected to the pneumatic cylinder 132 such that the cylinder rod 134 can rectilinearly move. The lower end of the cylinder rod 134 is connected to the rod portion 27 of the piston 20 of the solution vial. As shown in FIG. 11, a pair of branched hooks 135 are formed on the lower end of the cylinder rod portion 27, and are snap-connected to the through hole of the rod portion 27 of the piston 20. The cylinder rod 134 of the piston operating unit 130 is made of synthetic resin, thus having flexibility.

The blocks 112 and 113 of the solution vial operating unit 110 and the pneumatic cylinder 132 of the piston operating unit 130 are integrally fixed to a pair of the connection rods 136, which are formed through both sides thereof. The lowermost block 115 of the solution vial operating unit 110 is elastically fixed to the lower block 113 of the two-step blocks 112 and 113 by the spring (not shown).

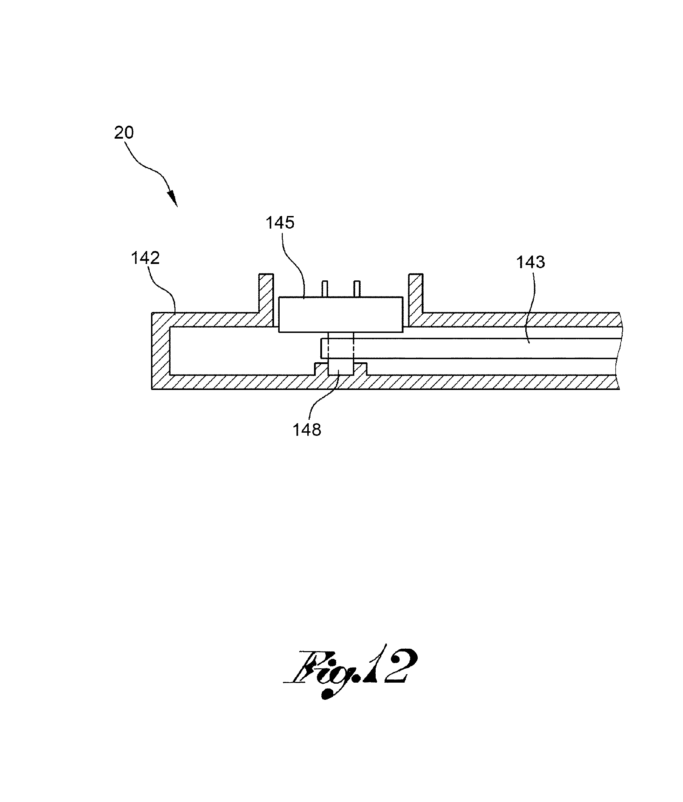

FIG. 12 is a schematic longitudinal sectional view of the lid open unit 140 of the tumor screening system of the present invention. As shown in FIGS. 8, 9, and 12, each of the lid open units 140 comprises a moving portion 142 installed below the solution vial operating unit 110 and moving back and forth, and a lid rotator 145 installed on the upper surface of the moving portion 142. The lid rotator 145 has a depressed structure so that the lid 40 is laid on the upper surface of the lid rotator 145, three connection protrusions 146, which are inserted into the connector insertion holes 42 of the lid 40, are formed on the center of the lid rotator 145, and a rotary shaft 148 extended downwardly from the lid rotator 145 is contained in the moving portion 142 and connected to a chain 143 driven by a motor.

As shown in FIG. 9, each of the filter operating units 150 comprises a pneumatic cylinder 155, and clamps 152 moved by the pneumatic cylinder 155 for clamping the filter 50. The clamps 152 clamp the filter 50 picked up from a filter loader having a plurality of the filters 50 loaded therein, and carry the filter 50 to a standby position.

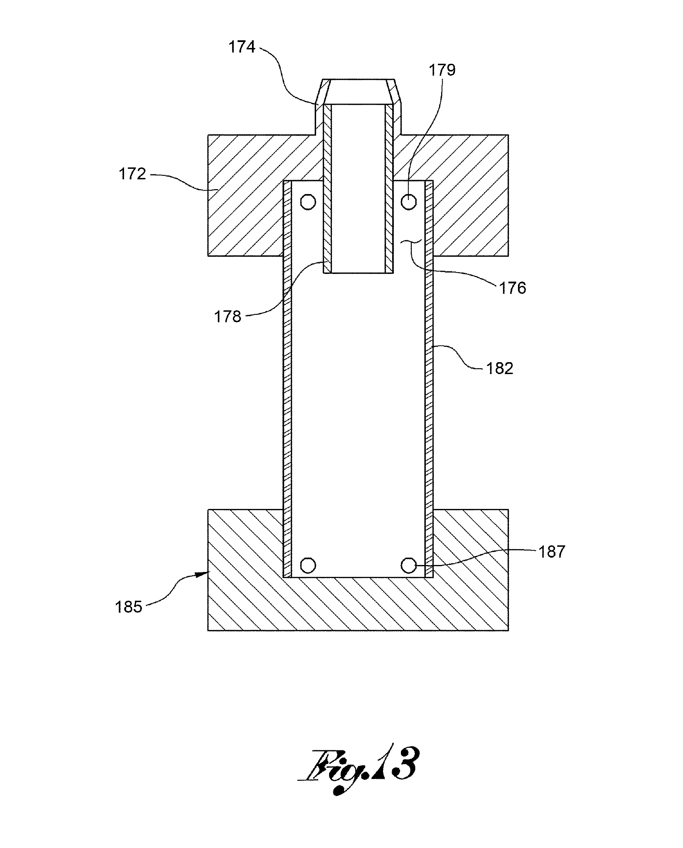

FIG. 13 is a partial side view of the slide operating unit of the tumor screening system of the present invention.

As shown in FIGS. 8, 9, and 13, the slide operating units 160 move between the filter standby position and a slide drop position to cause contact between the slide and the filter 50, and drops the slide contacting the filter 50 into a solution containing coagulated cells. Each of the slide operating units 160 comprises a moving portion 161 connected to a cylinder rod 169 operated by a pneumatic cylinder 165, a rotating portion 163 rotatably hinged to the moving portion 161, and a slide mounting portion 167 elastically fixed to the lower part of the rotating portion 163. The moving portion 161 moves along the linear guide 119, the rotating portion 163 is provided with the pneumatic cylinder 165, and the slide mounting portion 167 is connected to the cylinder rod 169 connected to the pneumatic cylinder 165. The rotating portion 163 includes a rectangular plane extended from the hinged portion thereof, and is connected to a motor, thus being rotatable in a designated angle range. The slide mounting portion 167 is elastically fixed to the rectangular plane via bolts 168, each being provided with a spring. A guide groove is formed in the lower surface of the slide mounting portion 167 so that the slide can be inserted into the guide groove.

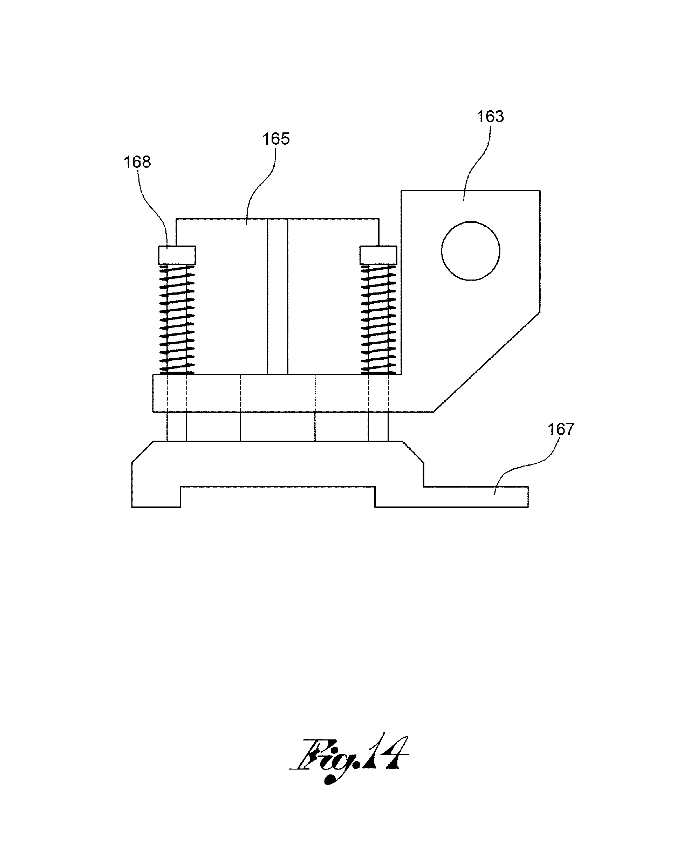

FIG. 14 is a schematic sectional view of the suction unit of the tumor screening system of the present invention.

As shown in FIGS. 8, 9, and 14, the suction units 170 suck the mixed solution in the solution vial via the filter 50. Each of the suction unit 170 comprises a filter connecting portion 172 having a hollow structure and connected to the lower portion of the filter 50 under the collection film 51, a solution receiving tube 182 connected to the lower portion of the hollow of the filter connecting portion 172 for guiding the solution downwardly, a solution receiving tube supporter 185 connected to the lower end of the light receiving tube 182 and having solution discharge holes 187 for discharging the solution, and a moving block to which the filter connecting portion 172 and the solution receiving tube supporter 185 are fixed. The moving block is connected to a pneumatic cylinder unit so that the moving block can move along the linear guide 119. An interference fit portion 174 connected to the cylindrical connection groove 58 of the filter 50 is formed on the upper surface of the filter connecting portion 172, an inner space 176, the lower end surface of which is opened, is formed in the filter connecting portion 172, and a solution guide nozzle 178 is connected to the lower portion of the interference fit portion 174 and is extended downwardly via the inner space 176. The inner space 176 of the filter connecting portion 172 is connected to suction holes 179 for sucking air in the inner space 176. The suction holes 179 are connected to a sucking instrument (not shown) having a similar structure to that of an injector. The solution receiving tube supporter 185 has an inner space connected to the solution receiving tube 182, and the solution discharge holes 187, which are selectively opened and closed to discharge the solution to the outside, are formed through one side of the inner space. The solution discharge holes 187 may discharge the solution to a reservoir installed in the system.

As shown in FIG. 9, each of the alcohol receiving units 200 receives alcohol for coagulate cells of the slide, and the upper portion of each of the alcohol receiving units 200 is opened so that the dropped slide is dipped into the alcohol therethrough.

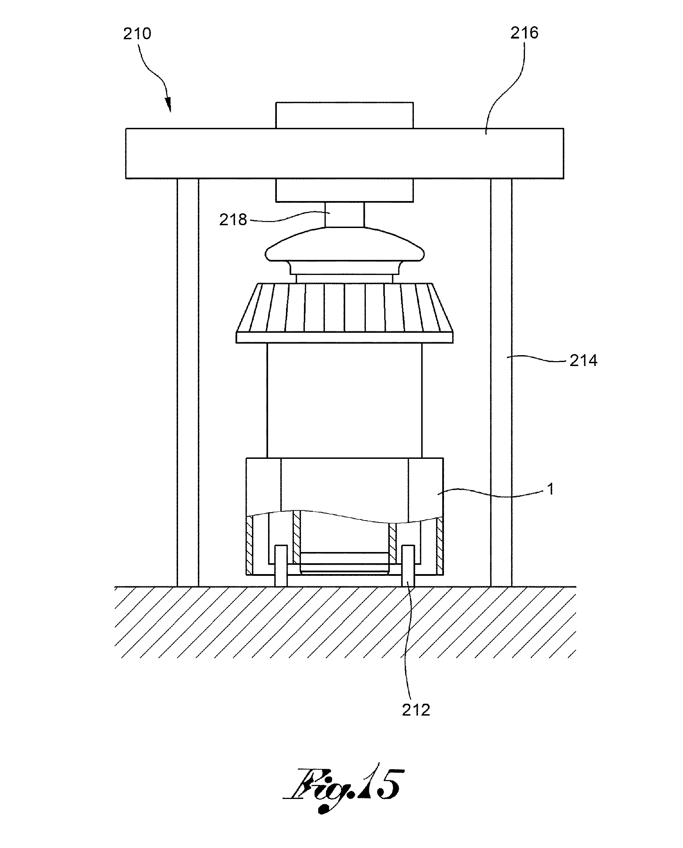

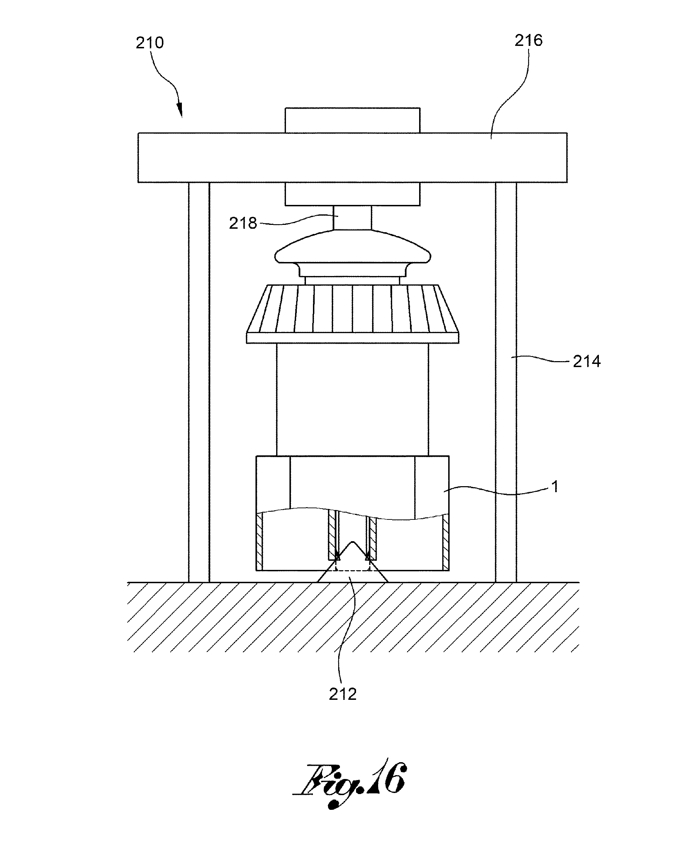

FIG. 15 is a schematic front view illustrating the connection of the solution vial to the piston connection releasing unit of the tumor screening system in accordance with the present invention, and FIG. 16 is a schematic side view illustrating the connection of the solution vial to the piston connection releasing unit of the tumor screening system in accordance with the present invention.

As shown in FIGS. 1, 15 and 16, the piston connection releasing units 210 release the snap connection of the guide slit 8 of the solution vial and the rod portion 27 of the piston 20. Each of the piston connection releasing units 210 comprises a pair of triangular protrusions 212 formed on a table, and a pneumatic cylinder unit installed above the triangular protrusions 212 through connection rods 214. Lower portions of the triangular protrusions 212 have a width larger than the interval between the first planes 9 of the guide slit 8. The pneumatic cylinder unit comprises a pneumatic cylinder 216 fixed to the connection rods 214, and a cylinder rod 218 pressing the solution vial.

The tumor screening system of the present invention further comprises LCD panels 222 for displaying the operations of the above pneumatic cylinders and the positions of the moving members according to the operations.

Hereinafter, a process for mixing cells with a solution using the solution vial of the present invention and transferring the cells from the solution vial to a slide by the tumor screening system of the present invention will be described.

First, a step of putting a cell collected member into the solution vial in order to mix collected cells with the solution is performed.

A user separates the gauze film installation unit 30, to which the lid 40 is connected, from the casing 1 by turning the gauze film installation unit 30 by hand, and injects a solution for soaking cells into the casing 1. Thereafter, the user puts the cell collected member into the casing 1, and connects the gauze film installation unit 30 to the casing 1.

Second, a step of installing the solution vial having the cell collected member and transferring the collected cells from the solution vial to a slide is performed.

The receiving portion 105 of the mixed solution forming unit 100 is opened, and the solution vial having the cell collected member is installed in the receiving portion 105. More specifically, the lower portion of the casing 1 is connected to protrusions of a rotating plate formed in the receiving portion 105, and the receiving portion 105 having the solution vial is pushed into the agitating chamber 102. The rotating plate is rotated at a predetermined speed for a predetermined time using a control unit. When the solution vial is rotated, the agitating protrusions 5 of the solution agitating portion 4 of the casing 1 and the impellers 24 of the piston 20 stir the solution in the solution vial, and the cells are separated from the cell collected member and are mixed with the solution in the solution vial.

The solution vial, in which the cells and the solution are sufficiently mixed with each other, is inserted into a space between the two-step blocks 112 and 113 under the condition that the lowermost block 115 of the solution vial operating unit 110 is lowered to a designated distance by pressing, and, when the force pressing the lowermost block 115 is eliminated, the lowermost block 115 is elevated by the restoring force of the spring, and the lowermost block 115 pushes the solution vial upwardly under the condition that the gauze film installation unit 30 of the solution vial is caught by the hollow of the lowermost block 115, thus fixing the solution vial to the solution vial operating unit 110. When the pneumatic cylinder 132 of the piston operating unit 130 is operated so that the cylinder rod 134 is lowered under the above condition, the branched hooks 135 formed on the lower end of the cylinder rod portion 27 are snap-connected to the through hole of the rod portion 27 of the piston 20. Accordingly, the connection between the piston 20 of the solution vial and the piston operating unit 130 under the condition that the solution vial is fixed to the solution vial operating unit 110 is completed.

Thereafter, the clamps of the filter operating unit 150 clamp the filter 50 slid out of the filter loader, transfers it to a position above the suction unit 170, and stops.

Thereafter, when the moving portion 142 of the lid open unit 140 moves forwardly, the lid rotator 145 is located below the lid 40 of the solution vial. Then, when the pneumatic cylinder 120 of the solution vial operating unit 110 is operated so that the moving block 118 is lowered, the two-step blocks 112 and 113 integrally fixed to the moving block 118 are lowered and the solution vial is lowered also. Here, the protrusions 146 of the lid rotator 145 are inserted into the connector insertion holes 42 of the lid 40 of the solution vial, and when the motor is controlled so that the lid rotator 145 is rotated, the screw connection between the lid 40 and the gauze film installation unit 30 is released so that the lid 40 is separated from the gauze film installation unit 30, and the pneumatic cylinder 120 is operated so that the moving block 118 of the solution vial operating unit 110 is elevated to a distance corresponding to the height of the lid 40, thereby allowing the lid 40 to be smoothly opened. When the pneumatic cylinder 132 of the piston operating unit 130 is operated together with the opening of the lid 40 so that the cylinder rod 134 is elevated, the piston 20 connected to the end of the cylinder rod 134 is elevated to lower the internal pressure of the casing 1 of the solution vial, thereby preventing the solution from pouring out of the solution vial. The moving portion 142 of the lid open unit 140, on which the separated lid 40 is laid, moves backwardly.

When the pneumatic cylinder 120 of the solution vial operating unit 110 is operated under the condition that the lid 40 of the solution vial is opened, the moving block 118 is lowered, and the connection flange 54 on the upper surface of the filter 50 is connected to a space between the outer wall 34 and the inner connection wall 35 of the gauze film installation unit 30 by interference fit, and simultaneously, when the pneumatic cylinder of the suction unit 170 is operated so that the moving block of the suction unit 170 is elevated, the interference fit portion 174 on the upper surface of the filter connecting portion 172 is connected to the cylindrical connection groove 58 formed in the lower surface of the filter 50 by interference fit. Accordingly, the solution vial, the filter 50, and the suction unit 170 are connected in a hermetically sealed state. Since the filter 50 is supported by the clamps of the filter operating unit 150, the solution vial and the suction unit 170 are simultaneously connected to the filter 50.

Thereafter, when the sucking instrument connected to the filter connecting portion 172 is operated under the condition that the solution discharge holes 187 formed through the solution receiving tube supporter 185 of the suction unit 170 are closed, the pressure in the filter connecting portion 172 and the pressure in the solution receiving tube 182 are lowered, and the mixed solution is sucked through the gauze film 32 and is filtered by the collection film 51 of the filter 50. The solution without cells flows toward the solution receiving tube 182. The amount of the mixed solution sucked from the solution vial is adjusted by controlling the sucking instrument. When the solution is discharged from the solution vial, the pneumatic cylinder 132 of the piston operating unit 130 is not operated and the piston 20 freely descends.

Thereafter, when the pneumatic cylinder 120 of the solution vial operating unit 110 is operated to elevate the moving block 118 and the pneumatic cylinder 132 of the piston operating unit 130 is operated to elevate the piston 20 of the solution vial, the solution vial is located on the filter 50 and the pressure in the casing 1 is not lowered, thereby preventing the mixed solution in the solution vial from pouring out downward.

Thereafter, the lid open unit 140 moves forwardly again under the condition that the solution vial is elevated, and a process opposite to the process for opening the lid 40 is performed, thus closing the solution vial with the lid 40.

When the pneumatic cylinder 132 of the piston operating unit 130 is operated to elevate the cylinder rod 134 to be distant from the solution vial closed with the lid 40, the piston 20 caught by the end of the cylinder rod 134 is elevated, and the connection grooves 29 of the rod portion 27 of the piston 20 are snap-connected to the fixing protrusions 10 of the guide slit 8 of the casing 1. Since the contact portion 22 of the piston 20 contacts the upper portion of the casing 1 under the snap-connected state, when the cylinder rod 134 is continuously elevated, the end of the cylinder rod 134 is slidably separated from the rod portion 27 of the piston 20. Since the piston 20 is forcibly drawn from the solution vial closed with the lid 40, the inside of the casing 1 becomes a vacuum state.

Thereafter, the solution vial is taken out of the solution vial operating unit 110 under the condition that the lowermost block 115 of the solution vial operating unit 110 is pressed downwardly.

Thereafter, when the sucking instrument of the suction unit 170 is additionally operated under the condition that the solution vial is separated from the filter 50, the solution remaining on the collection film 51 of the filter 50 is sucked into the lower portion of the system.

Thereafter, the pneumatic cylinder 165 connected to the moving portion 161 is operated to move the slide operating unit 160 on the filter 50 and the pneumatic cylinder connected to the slide mounting portion 167 is operated to lower the slide mounting portion 167 to the filter 50, thereby causing a slide to contact the collection film 51 of the filter 50. When the moving portion 161 is horizontally moved under the condition that the slide contacts the filter 50, the slide repeatedly contacts the collection film 51.

The slide receives cells from the collection film 51 of the filter 50, and is transferred to be above the alcohol receiving unit 200. Then, when the rotating portion 163 is rotated at an angle of 90 , the linear guide groove is perpendicularly erected and the slide is separated from the guide groove and dropped into the alcohol receiving unit 200.

The slide, which is soaked in alcohol, is taken out of the alcohol receiving unit 200, and is used to test the cells thereon.

After testing, the solution vial, which is stored under the condition that the inside of the casing 1 is in the vacuum state, may be used again. In this case, the piston connection releasing unit 210 releases the snap-connection between the piston 20 and the casing 1. More specifically, when the pneumatic cylinder 216 is operated under the condition that the solution vial is located at a position so that the triangular protrusions 212 of the piston connection releasing unit 210 are inserted into a space between the first planes 9 having the fixing protrusions 10 of the guide slit 8 of the solution vial, and the solution vial is pressed downwardly, the space between the first planes 9 is widened by the triangular protrusions 212, thereby releasing the snap-connection between the piston 20 and the casing 1 and allowing the piston 20 to descend to a position corresponding to the amount of the remaining solution in the solution vial. That is, the piston 20 is returned to a state freely moving against the solution vial.

The solution vial of the present invention serves as not only a container for containing a cell sample, mixing cells with a solution, and storing a mixed solution, but alto to be directly installed in the tumor screening system and discharge the solution downwardly.

Further, since the solution vial of the present invention has agitating units, when the solution vial is rotated, the solution in the solution vial is agitated. By rotating the solution vial for a predetermined agitating time, the uniform mixing of the cells and the solution is achieved.

Since the piston 20 moves, the solution vial of the present invention discharges the solution under the condition that the solution in the solution vial is isolated from external air, and thus, the external air is not supplied to the inside of the solution vial. Accordingly, the solution remaining in the solution vial is not deteriorated by air, and is able to be used later.

When the lid 40 of the solution vial is opened, the piston 20 is elevated to a small height so that the pressure in the casing 1 is lowered, and the solution of the casing 1 does not pour through the gauze film 32 due to a suction effect resulting from a difference of pressures. Thereby, it is possible to minimize a loss of the solution and to prevent the filter 50 from being contaminated by the pouring solution.

Since the solution vial of the present invention preserves the mixed solution under the condition that the rod portion 27 of the piston 20 is snap-connected to the upper end of the guide slit 8 of the casing 1, the inside of the solution vial is vacuumed, thus preventing spoilage of the solution in the solution vial.

When the solution vial of the present invention is inserted into the mixed solution forming unit 100, the solution vial is rotated so that cells are uniformly mixed with the solution by the agitating protrusions and the impellers of the solution vial.

When the mixed solution is discharged from the solution vial, the piston 20 freely descends and thus it is possible to discharge the mixed solution from the solution vial in a hermetically sealed state.

Since the lid open unit opens and closes the lid of the solution vial, a process time is reduced.

Since the solution guide nozzle formed in the filter connecting portion is located under the suction holes, although the solution is sucked into the solution vial due to decrease in the pressure in the solution vial, it is possible to prevent the solution from flowing into the suction holes.

Since a user does not participate in a process for transferring the cells from the collection film of the filter to the slide using the slide operating unit and dropping the slide having the cells into alcohol, it is possible to prevent the slide from being contaminated by user's hand.

Since the piston connection releasing unit releases the snap-connection of the piston and the solution vial, the solution vial can be rapidly reused.

The tumor screening system of the present invention reuses the remaining mixed solution in the solution vial without use of a new solution vial, thus reducing the amount of waste generated.

Since all operating states of the tumor screening system of the present invention are displayed on the LCD panels, the tumor screening system can be conveniently controlled.

Although the preferred embodiment of the present invention has been disclosed for illustrative purposes, those skilled in the art will appreciate that various modifications, additions and substitutions are possible, without departing from the scope and spirit of the invention as disclosed in the accompanying claims. Hereinafter, a brush for liquid based cytology of cervix carcinoma in accordance with a preferred embodiment of the present invention will be described in detail with reference to the annexed drawings.

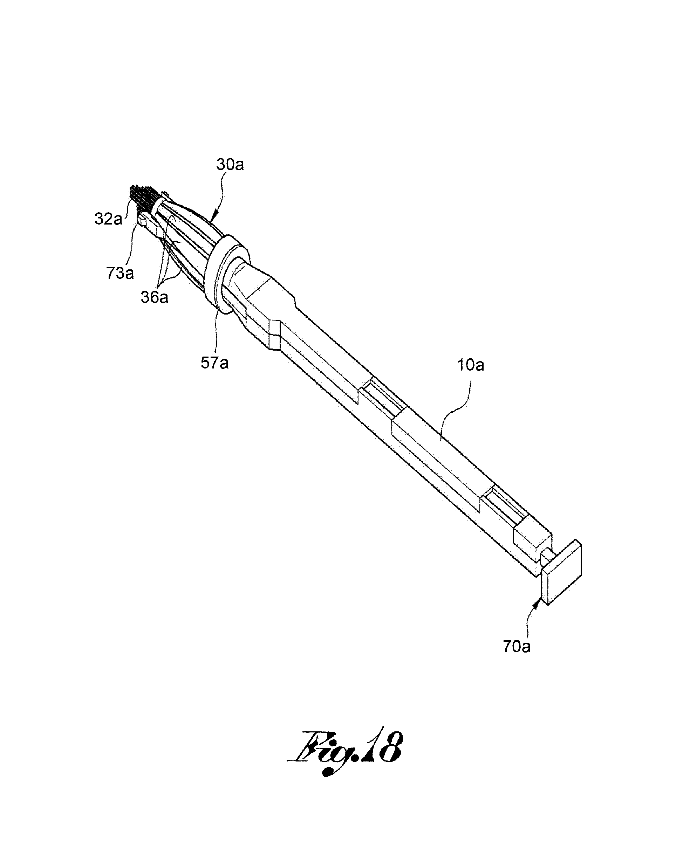

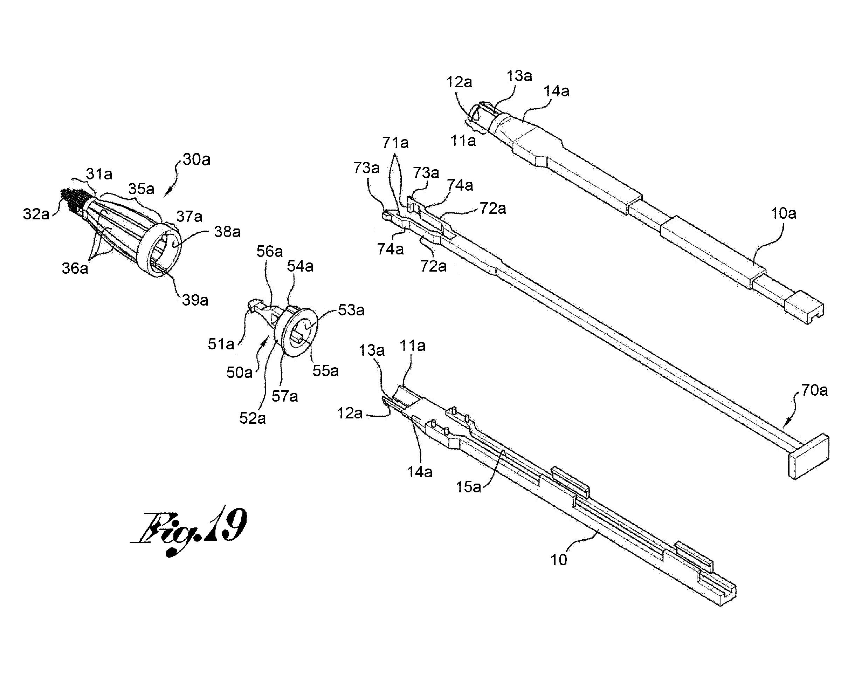

As shown in FIGS. 18 to 20, the brush for liquid based cytology of the present invention comprises a holding unit 10, a cell collecting member 30, an inner detachable member 50, and a movable member 70. Here, the direction of the cell collecting member 30, i.e., the direction of the cell collecting member 30, which is inserted into a woman's genital organ, is referred to as a first direction, and a direction opposite to the first direction is referred to as a second direction.

The holding unit 10 has a cylindrical rod structure having a sliding space 15 formed therein. A designated portion of the holding unit 10 may be inserted into a woman's genital organ, when an operator collects cells from a woman's uterus.

The holding unit 10 comprises an insertion portion 11 having a protrusion 12 formed thereon. The insertion portion 11 is formed at one end of the holding unit 10 in the first direction, and has an approximately circular cross section.

The protrusion 12 formed at the end of the insertion portion 11 in the first direction is extended outwardly from the end of the insertion portion 11. Here, the protrusion 12 is caught by or released from a second connection portion 52 of the inner detachable member 50, thereby allowing the inner detachable member 50 to be detachably connected to the holding unit 10.

The cell collecting member 30 collects cells from various regions of cervix, such as an inner wall and a transvaginal portion of cervix, when the cell collecting member 30 is inserted into a woman's genital organ. The cell collecting member 30 comprises a first collecting unit 31, a second collecting unit 35, and a first connecting unit 37.

The first collecting unit 31 comprises a plurality of collecting protrusions 32, a first hook-latched portion 33, and second hook-latched portions 34. The collecting protrusions 32 of the first collecting unit 31 collect cells from the inner wall and the transvaginal portion of cervix, when the cell collecting member 30 is inserted into the woman's genital organ.

A first hook 51 of the inner detachable member 50, which will be described later, is latched onto and released from the first hook-latched portion 33. Here, the first hook 51 of the inner detachable member 50 is inserted into and separated from the first hook-latched portion 33 in the lengthwise direction of the holding portion 10.

Further, second hooks 71 of the movable member 70 are latched onto and released from the second hook-latched portions 34. Here, the second hooks 71 of the movable member 70 are latched centripetally onto and released from the second hook-latched portions 34.

The second collecting unit 35 is extended from the first collecting unit 31 in the lengthwise direction of the holding unit 10. Here, the second collecting unit 35 elastically expands and contracts in the lengthwise direction. Thus, when the second collecting unit 35 contracts, the second collecting unit 35 causes the first collecting unit 31 to approach the first connecting unit 37.

The first connecting unit 37 has an approximately cylindrical structure having an insertion hole 38, into which a second connecting unit 52 of the inner detachable member 50 is inserted.

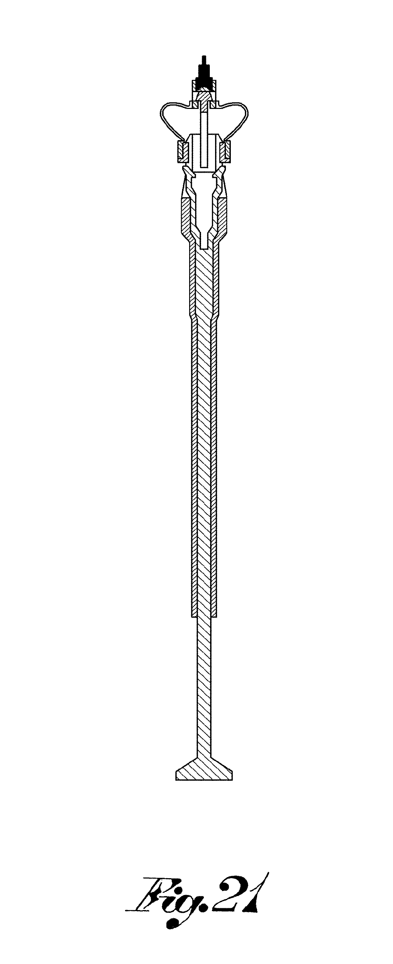

The second collecting unit 35 has a plurality of collecting combs 36, which are separated from each other by a designated interval along the circumferential direction centering on an axis of the holding unit 10 in the lengthwise direction. The collecting combs 36 connect the first collecting unit 31 and the first connecting unit 37 to each other, and are elastically bent and contract radially towards the outside of the axis of the holding unit 10 in the lengthwise direction (with reference to FIG. 21), when the first collecting unit 31 approaches the first connecting unit 37 by the sliding of the movable member 70, which will be described later. In case that the operator rotates the brush 1 of the present invention along the axis of the holding unit 10 in the lengthwise direction under the condition that the collecting combs 36 of the second collecting unit 35 are elastically bent, the collecting combs 36 easily collect cells from the transvaginal portion of cervix.

The inner detachable member 50 comprises the second connecting unit 52, the first hook 51, and a connecting member 56.

As described above, the first hook 51 is latched onto and released from the first hook-latched portion 33 of the first collecting unit 31 of the cell collecting member 30. When the first hook 51 is latched onto the first hook-latched portion 33, the cell collecting member 30 and the inner detachable member 50 move together.

The second connecting unit 52 is latched onto and released from the protrusion 12 formed on the insertion portion 11 of the holding unit 10, thereby allowing the inner detachable member 50 to be detachably attached to the insertion portion 11 of the holding unit 10. The second connecting unit 52 has an approximately cylindrical structure having a through hole 53 so that the movable member 70 passes through the through hole 53 in the lengthwise direction of the holding unit 10.

The second connecting unit 52 is inserted into the insertion hole 38 of the first connecting unit 37 of the cell collecting member 30, thus being connected to the cell collecting member 30. At least one first guide rib 54 protruded outwardly from the second connecting unit 52 in the lengthwise direction of the holding unit 10 is formed on the outer wall of the second connecting unit 52. Correspondingly, a first guide groove 39, into which the first guide rib 54 is inserted, when the second connecting unit 52 is inserted into the insertion hole 38 of the first connecting unit 37, is formed in the inner wall of the insertion hole 38 of the first connecting unit 37 of the cell collecting member 30. Thereby, it is possible to stably insert the second connecting unit 52 into the first connecting unit 37 and prevent the cell collecting member 30 and the inner detachable member 50 from independently rotating. Otherwise, the first guide groove 39 may be formed in the outer wall of the second connecting unit 52, and the first guide rib 54 may be formed on the inner wall of the first connecting unit 37.

The connecting member 56 connects the first hook 51 and the second connecting unit 52 to each other. The connecting member 56 has an approximately V beam structure, both ends of which are connected to the second connecting unit 52. The first hook 51 is formed on a valley portion of the V beam-structured connecting member 56 towards the first hook-latched portion 33 of the cell collecting member 30.

The movable member 70 comprises the second hooks 71 formed at one end thereof in the first direction so that the second hooks 71 are latched onto and released from the second hook-latched portions 34 of the cell collecting member 30. The movable member 70 is received in the holding unit 10 so that the movable member 70 can slide between a first latched position and a second latched position.

Here, the first latched position denotes a position at which the second hooks 71 of the movable member 70 are latched onto the second hook-latched portions 34 of the cell collecting member 30, and the second latched position denotes a position at which the first hook 51 of the inner detachable member 50 is latched onto the first hook-latched portion 33 of the cell collecting member 30 according to the approach of the first collecting unit 31 to the first connecting unit 37 due to the contraction of the second collecting unit 35 under the condition that the second hooks 71 are latched onto the second hook-latched portions 34 .

That is, the collecting combs 36 of the second collecting unit 35 of the cell collecting member 30 are stretched at the first latched position, and the collecting combs 36 of the second collecting unit 35 of the cell collecting member 30 are radially bent at the second latched position. The inner detachable member 50 comprises a baffle flange 57 extended outwardly from the second connecting unit 52 for preventing the first connecting unit 37 from retreating to the holding unit 10 due to the elastic force of the second collecting unit 35 at the second latched position. Thereby, at the second latched position, the latching of the first hook 51 of the inner detachable member 50 onto the first hook-latched portion 33 of the cell collecting member 30 is maintained and the baffle flange 57 of the inner detachable member 50 prevents the retreat of the first connecting unit 37 of the cell collecting member 30, thus maintaining the radial bending of the collecting combs 36 of the cell collecting member 30, as shown in FIG. 21.

Extended branches 72 creating an approximately V beam shape are formed at the end of the movable member 70 in the first direction. The second hooks 71 of the movable member 70 are respectively protruded from both ends of the extended branches 72 towards the opposite ends. Thereby, as described above, when the movable member 70 enters from the first latched position, the second hooks 71 are latched centripetally onto the second hook-latched portions 34 formed at both sides of the first collecting unit 31.

A second guide rib 55 is formed on the inner wall of the second connecting unit 52 of the inner detachable member 50 in the lengthwise direction of the holding unit 10. Correspondingly, a second guide groove 13 for guiding the sliding of the second guide rib 55 when the insertion portion 11 is inserted into the second connecting unit 52 of the inner detachable member 50 is formed in the insertion portion 11 of the holding unit 10. Thereby, it is possible to stably insert the insertion portion 11 of the holding unit 10 into the second connecting unit 52 of the inner detachable member 50 when the holding unit 10 and the inner detachable member 50 are connected, and prevent the inner detachable member 50 and the holding unit 10 from independently rotating.

Further, third guide grooves 14 for guiding the sliding of the extended branches 72 of the movable member 70 in the lengthwise direction of the holding unit 10 are formed in the insertion portion 11 of the holding unit 10.

The second hooks 71 of the movable member 70 at the second latched position are released from the second hook-latched portions 34 of the cell collecting member 30, and pass through the through hole 53 of the second connecting unit 52 of the inner detachable member 50 from the first direction to the second direction, thereby allowing the movable member 70 to slidably move from the second latched position to a latch-releasing position in the sliding space 15 of the holding unit 10.

In case that the operator moves the movable member 70 from the latch-releasing position in the first direction, pressing portions 73 for pressing the end of the second connecting unit 52 of the inner detachable member 50 in the first direction are formed on the ends of the extended branches 72 of the movable member 70.

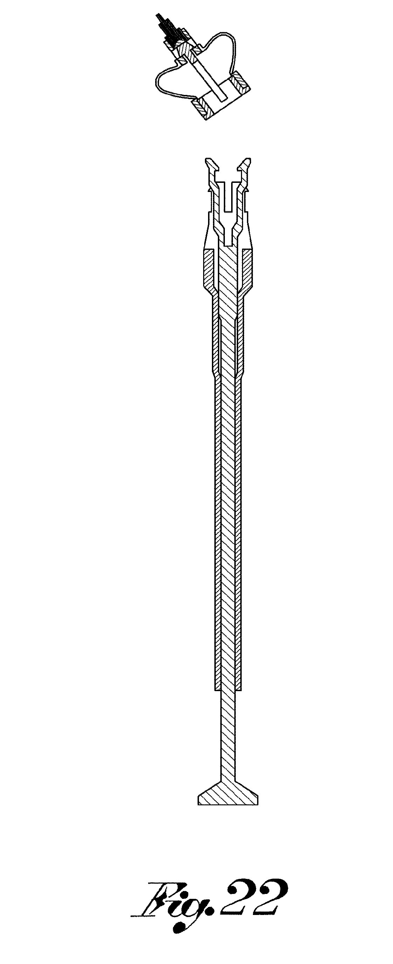

When the operator presses the movable member 70 at the latch-releasing position in the first direction at a force having at least a designated intensity, the protrusion 12 formed on the insertion portion 11 of the holding unit 10 is released from the second connecting unit 52 of the inner detachable member 50, thereby separating the inner detachable member 50, which is connected to the cell collecting member 30, from the holding unit 10. Thereby, after the collection of cells using the cell collecting member 30 is completed, the operator can separate the cell collecting member 30 from the holding unit 10 without using hand or other tools. At this time, since the latching of the first hook 51 of the inner detachable member 50 onto the first hook-latched portion 33 of the cell collecting member 30 is maintained and the baffle flange 57 of the inner detachable member 50 prevents the retreat of the first connecting unit 37 of the cell collecting member 30, as described above, the inner detachable member 50 and the cell collecting member 30 can be separated from the holding unit 10 while maintaining their shapes, as shown in FIG. 22.

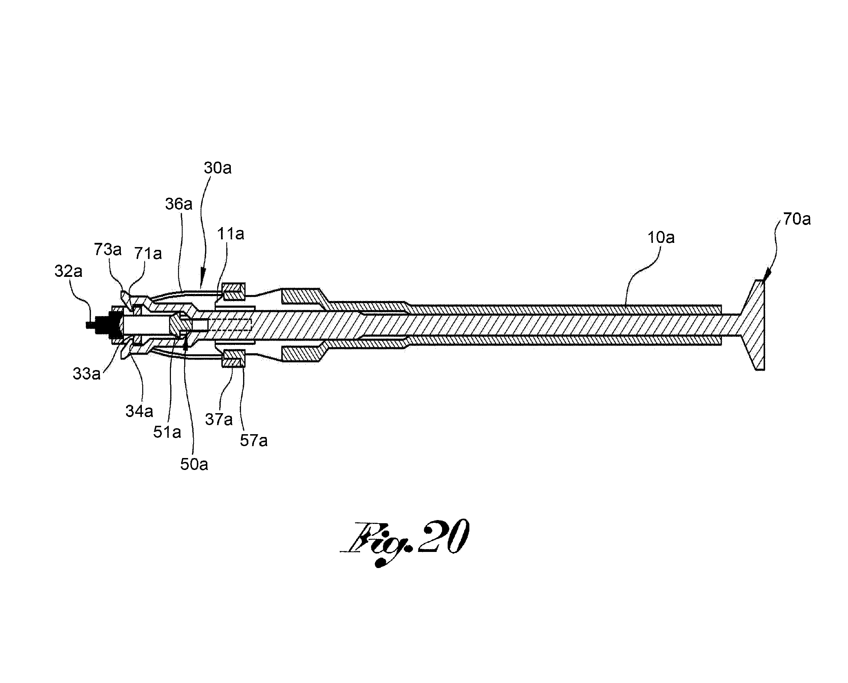

Hereinafter, with reference to FIGS. 20 to 22, a process for collecting cells from cervix using the brush of the present invention will be described in detail.

Before collecting cells from cervix, the movable member 70 of the brush 1 of the present invention is maintained at the first latched position, as shown in FIG. 20. Otherwise, the movable member 70 may be maintained at a position prior to the first latched position, or an operator may press the movable member 70 in the first direction so that the movable member 70 enters the first latched position. In this case, when the movable member 70 is maintained at the position prior to the first latched position, the end of the movable member 70 in the first direction, i.e., the end of the movable member 70 having the second hooks 71, is located between the second hook-latched portions 34 of the cell collecting member 30 and the second connecting unit 52 of the inner detachable member 50.

At the first latched position, the insertion portion 11 of the holding unit 10 is inserted into the through hole 53 of the second connecting unit 52 of the inner detachable member 50 so that the latching of the protrusion 12 onto the second connecting unit 52 of the inner detachable member 50 is maintained, and the second connecting unit 52 of the inner detachable member 50 is inserted into the first connecting unit 37 of the cell collecting member 30.

Thereafter, the operator inserts the brush 1 of the present invention, in which the movable member 70 is located at the first latched position, into a woman's genital organ. Since the brush 1 is inserted into the woman's genital organ under the condition that the second collecting unit 35 of the cell collecting member 30 is stretched, as shown in FIG. 20, the brush 1 can be inserted into the woman's genital organ, i.e., cervix, without damage to the virginal wall.

Thereafter, the operator pulls a portion of the movable member 70, which is exposed from the end of the holding unit 10 in the second direction, in the second direction so that the movable member 70 slidably moves from the first latched position to the second latched position. That is, the first collecting unit 31 of the cell collecting member 30 approaches the first connecting unit 37 by the elastic contraction of the second collecting unit 35, and during the approach of the first collecting unit 31 to the first connecting unit 37, the movable member 70 enters the second latched position in which the first hook 51 of the inner detachable member 50 is latched onto the first hook-latched portion 33 of the cell collecting member 30. At this time, the second collecting unit 35 of the cell collecting unit 30 is radially bent, as shown in FIG. 21.

Thereafter, the operator inserts the first collecting unit 31 into the cervix under the condition the movable member 70 is located at the second latched position. In this case, the second collecting unit 35 in the radially bent state is not inserted into the cervix, but contacts the inner wall of a vaginal part of the transvaginal portion of the cervix.

Then, the operator rotates the brush 1 under the condition that the second collecting unit 35 is inserted into the cervix and the second collecting unit 35 contacts the inner wall of the vaginal part of the transvaginal portion of the cervix, thereby causing the first collecting unit 31 to collect cells from the inside of the cervix and the rotating collecting combs 36 of the second collecting unit 35 to collect cells from the inner wall of the vaginal part of the transvaginal portion of the cervix. Thereby, it is possible to collect cells from various regions of the cervix, such as the inside of the cervix and the transvaginal portion of the cervix.

After the collection of the cells is completed, the operator pulls the brush 1 out of the woman's uterus. Thereafter, the operator pulls the portion of the movable member 70, which is exposed from the end of the holding unit 10 in the second direction, in the second direction by applying a force having at least a designated intensity, thus moving the movable member 70 to the latch-releasing position.

When the operator pulls the movable member 70 in the second direction by applying the force having at least the designated intensity, the second hooks 71 of the movable member 70 are released from the second hook-latched portions 34 of the first collecting unit 31, pass through the through hole 53 of the second connecting unit 52 of the inner detachable member 50, and are transferred to the holding unit 10.

In this case, when the movable member 70 moves to the latch-releasing position, at least portions of the extended branches 72 of the movable member 70 contact the third guide grooves 14 of the holding unit 10, and thus the extended branches 72 approach each other. Preferably, inclined planes 74 are formed on at least portions of side surfaces of the extended branches 72 so that the inclined planes 74 contact the inner walls of the third guide grooves 14 and thus the extended branches 72 approach each other. Thereby, the extended branches 72 of the movable member 70 easily pass through the through hole 53 of the inner detachable member 50.

Thereafter, the operator pushes the portion of the movable member 70, which is exposed from the end of the holding unit 10 in the second direction, in the first direction so that the pressing portions 73 of the movable member 70 press the second connecting unit 52 of the inner detachable member 50, thereby causing the protrusion 12 of the insertion portion 11 of the holding unit 10 to be released from the second connecting unit 52 of the inner detachable member 50, and thus separating the inner detachable member 50 from the holding unit 10. Thereby, the inner detachable member 50, to which the cell collecting member 30 is connected, is separated from the holding unit 10. When the inner detachable member 50 is separated from the holding unit 10, the operator locates the cell collecting member 30 and the inner detachable member 50 at an inlet of a container, and pushes the movable member 70 in the first direction, thus allowing the cell collecting member 30 and the inner detachable member 50 to be put into the container. Accordingly, a separate device or operator's hands for putting the cell collecting member 30 and the inner detachable member 50 into the container is/are not required. Thereby, it is possible to stably and precisely collect and diagnose cells.

Another cell collecting member 30 and another inner detachable member 50 may be connected to the holding unit 10 and the movable member 70, from which the cell collecting member 30 and the inner detachable member 50 are removed.

Hereinafter, a supporting solution for cytodiagnosis in accordance with a preferred embodiment of the present invention will be described in detail.

The supporting solution for cytodiagnosis of the present invention comprises a buffer agent for storing collected cells under the condition that the acidity of the collected cells is maintained in a designated pH range, alcohol for suppressing the protein degeneration of the stored cells to maintain the pathological structures of the cells, an anti-desiccant for preventing the stored cells from drying during subsequent cytopathological tests, and a preservative for preventing the stored cells from rotting.

The supporting solution for cytodiagnosis of the present invention stores the collected cells while maintaining the morphologic structures of the collected cells, allows the pathological test of the stored cells to be easily and precisely performed, facilitates the DNA extraction from the stored cells, and is nonflammable and thus is easy to handle.

Various pharmaceuticals, which were used in various examples of the present invention, are products of SIGMA. Supporting solutions of the various examples were manufactured by mixing and agitating a buffer agent, alcohol, and an anti-desiccant, adding a preservative to the mixture (here, if necessary, the preservative is not added to the mixture according to the examples), and then adding distilled water to the mixture having the preservative for satisfying the weight ratio.

### [Example 1]

A supporting solution for cytodiagnosis containing 20 parts by weight of lactic acid with pH 3 as a buffer agent, 30 parts by weight of ethanol, 10 parts by weight of ethylene glycol, and 40 parts by weight of distilled water was manufactured.

### [Example 2]

A supporting solution for cytodiagnosis containing 20 parts by weight of lactic acid with pH 3 as a buffer agent, 30 parts by weight of ethanol, 10 parts by weight of ethylene glycol, 4 parts by weight of sorbic acid, and 36 parts by weight of distilled water was manufactured.

### [Example 3]

A supporting solution for cytodiagnosis containing 20 parts by weight of lactic acid with pH 3 as a buffer agent, 30 parts by weight of ethanol, 10 parts by weight of glycerol, 2 parts by weight of sorbic acid, 2 parts by weight of citric acid, and 36 parts by weight of distilled water was manufactured.

### [Example 4]

A supporting solution for cytodiagnosis containing 20 parts by weight of lactic acid with pH 3 as a buffer agent, 15 parts by weight of ethanol, 15 parts by weight of polyethylene glycol having a molecular weight of 2,834Da, 2 parts by weight of citric acid, 2 parts by weight of salicylic acid, and 36 parts by weight of distilled water was manufactured.

### [Example 5]

A supporting solution for cytodiagnosis containing 20 parts by weight of lactic acid with pH 3 as a buffer agent, 35 parts by weight of propanol, 10 parts by weight of ethylene glycol, and 35 parts by weight of distilled water was manufactured.

### [Example 6]

A supporting solution for cytodiagnosis containing 20 parts by weight of lactic acid with pH 3 as a buffer agent, 35 parts by weight of propanol, 10 parts by weight of ethylene glycol, 4 parts by weight of salicylic acid, and 31 parts by weight of distilled water was manufactured.

### [Example 7]

A supporting solution for cytodiagnosis containing 20 parts by weight of lactic acid with pH 3 as a buffer agent, 35 parts by weight of propanol, 5 parts by weight of glycerol, 2 parts by weight of formalin, 2 parts by weight of sodium benzoate, and 36 parts by weight of distilled water was manufactured.

### [Example 8]

A supporting solution for cytodiagnosis containing 20 parts by weight of lactic acid with pH 3 as a buffer agent, 35 parts by weight of propanol, 15 parts by weight of polypropylene glycol having a molecular weight of 1,854Da, 2 parts by weight of sorbic acid, 2 parts by weight of glyoxal, and 26 parts by weight of distilled water was manufactured.

### [Example 9]

A supporting solution for cytodiagnosis containing 15 parts by weight of lactic acid with pH 3 as a buffer agent, 35 parts by weight of isopropyl alcohol, 5 parts by weight of ethylene glycol, 4 parts by weight of sorbic acid, and 41 parts by weight of distilled water was manufactured.

### [Example 10]

A supporting solution for cytodiagnosis containing 15 parts by weight of lactic acid with pH 3 as a buffer agent, 35 parts by weight of isopropyl alcohol, 5 parts by weight of ethylene glycol, 2 parts by weight of sorbic acid, 2 parts by weight of citric acid, and 41 parts by weight of distilled water was manufactured.

### [Example 11]

A supporting solution for cytodiagnosis containing 15 parts by weight of lactic acid with pH 3 as a buffer agent, 35 parts by weight of isopropyl alcohol, 5 parts by weight of glycerol, 2 parts by weight of citric acid, 2 parts by weight of glyoxal, and 41 parts by weight of distilled water was manufactured.

### [Example 12]

A supporting solution for cytodiagnosis containing 15 parts by weight of lactic acid with pH 3 as a buffer agent, 35 parts by weight of isopropyl alcohol, 10 parts by weight of polyethylene glycol having a molecular weight of 2,834Da, 2 parts by weight of salicylic acid, 2 parts by weight of sodium benzoate, and 36 parts by weight of distilled water was manufactured.

### [Example 13]

A supporting solution for cytodiagnosis containing 20 parts by weight of acetic acid with pH 4 as a buffer agent, 30 parts by weight of ethanol, 5 parts by weight of ethylene glycol, and 45 parts by weight of distilled water was manufactured.

### [Example 14]

A supporting solution for cytodiagnosis containing 20 parts by weight of acetic acid with pH 4 as a buffer agent, 30 parts by weight of ethanol, 5 parts by weight of ethylene glycol, 2 parts by weight of ascorbic acid, and 43 parts by weight of distilled water was manufactured.

### [Example 15]

A supporting solution for cytodiagnosis containing 20 parts by weight of acetic acid with pH 4 as a buffer agent, 30 parts by weight of ethanol, 5 parts by weight of propylene glycol, 2 parts by weight of ascorbic acid, 2 parts by weight of sorbic acid, and 41 parts by weight of distilled water was manufactured.

### [Example 16]

A supporting solution for cytodiagnosis containing 20 parts by weight of acetic acid with pH 4 as a buffer agent, 30 parts by weight of ethanol, 5 parts by weight of propylene glycol, 2 parts by weight of sorbic acid, 2 parts by weight of citric acid, and 41 parts by weight of distilled water was manufactured.

### [Example 17]

A supporting solution for cytodiagnosis containing 20 parts by weight of acetic acid with pH 4 as a buffer agent, 30 parts by weight of ethanol, 5 parts by weight of polyethylene glycol having a molecular weight of 2,834Da, 2 parts by weight of citric acid, 2 parts by weight of glyoxal, and 41 parts by weight of distilled water was manufactured.

### [Example 18]

A supporting solution for cytodiagnosis containing 20 parts by weight of acetic acid with pH 4 as a buffer agent, 30 parts by weight of ethanol, 5 parts by weight of polypropylene glycol having a molecular weight of 1,854Da, 2 parts by weight of citric acid, 2 parts by weight of sodium benzoate, and 41 parts by weight of distilled water was manufactured.

### [Example 19]

A supporting solution for cytodiagnosis containing 20 parts by weight of acetic acid with pH 4 as a buffer agent, 35 parts by weight of propanol, 10 parts by weight of ethylene glycol, and 35 parts by weight of distilled water was manufactured.

### [Example 20]

A supporting solution for cytodiagnosis containing 20 parts by weight of acetic acid with pH 4 as a buffer agent, 35 parts by weight of propanol, 10 parts by weight of ethylene glycol, 2 parts by weight of formalin, and 33 parts by weight of distilled water was manufactured.

### [Example 21]

A supporting solution for cytodiagnosis containing 20 parts by weight of acetic acid with pH 4 as a buffer agent, 35 parts by weight of propanol, 5 parts by weight of glycerol, 2 parts by weight of formalin, 2 parts by weight of calcium propionate, and 36 parts by weight of distilled water was manufactured.

### [Example 22]

A supporting solution for cytodiagnosis containing 20 parts by weight of acetic acid with pH 4 as a buffer agent, 35 parts by weight of propanol, 10 parts by weight of polyethylene glycol having a molecular weight of 2,834Da, 2 parts by weight of citric acid, 2 parts by weight of ascorbic acid, and 31 parts by weight of distilled water was manufactured.

### [Example 23]

A supporting solution for cytodiagnosis containing 20 parts by weight of acetic acid with pH 4 as a buffer agent, 35 parts by weight of isopropyl alcohol, 10 parts by weight of ethylene glycol, 2 parts by weight of sorbic acid, and 33 parts by weight of distilled water was manufactured.

### [Example 24]

A supporting solution for cytodiagnosis containing 20 parts by weight of acetic acid with pH 4 as a buffer agent, 35 parts by weight of isopropyl alcohol, 10 parts by weight of ethylene glycol, 2 parts by weight of sorbic acid, 2 parts by weight of sodium benzoate, and 31 parts by weight of distilled water was manufactured.

### [Example 25]

A supporting solution for cytodiagnosis containing 20 parts by weight of acetic acid with pH 4 as a buffer agent, 35 parts by weight of isopropyl alcohol, 10 parts by weight of propylene glycol, 2 parts by weight of sorbic acid, 2 parts by weight of glyoxal, and 31 parts by weight of distilled water was manufactured.

### [Example 26]

A supporting solution for cytodiagnosis containing 20 parts by weight of acetic acid with pH 4 as a buffer agent, 35 parts by weight of isopropyl alcohol, 5 parts by weight of glycerol, 2 parts by weight of glyoxal, 2 parts by weight of citric acid, and 36 parts by weight of vapor was manufactured.

### [Example 27]

A supporting solution for cytodiagnosis containing 20 parts by weight of acetic acid with pH 4 as a buffer agent, 35 parts by weight of isopropyl alcohol, 10 parts by weight of polyethylene glycol having a molecular weight of 2,834Da, 2 parts by weight of salicylic acid, 2 parts by weight of citric acid, and 31 parts by weight of distilled water was manufactured.

### [Example 28]

A supporting solution for cytodiagnosis containing 20 parts by weight of phosphoric acid with pH 5 as a buffer agent, 20 parts by weight of ethanol, 10 parts by weight of ethylene glycol, 2 parts by weight of formalin, and 48 parts by weight of distilled water was manufactured.

### [Example 29]

A supporting solution for cytodiagnosis containing 20 parts by weight of phosphoric acid with pH 5 as a buffer agent, 30 parts by weight of propanol, 10 parts by weight of ethylene glycol, 2 parts by weight of sorbic acid, 2 parts by weight of salicylic acid, and 36 parts by weight of distilled water was manufactured.

### [Comparative Example 1]

An ethanol solution of 95% was used.

### [Comparative Example 2]

A PBS (Phosphate Buffered Saline) solution was used.

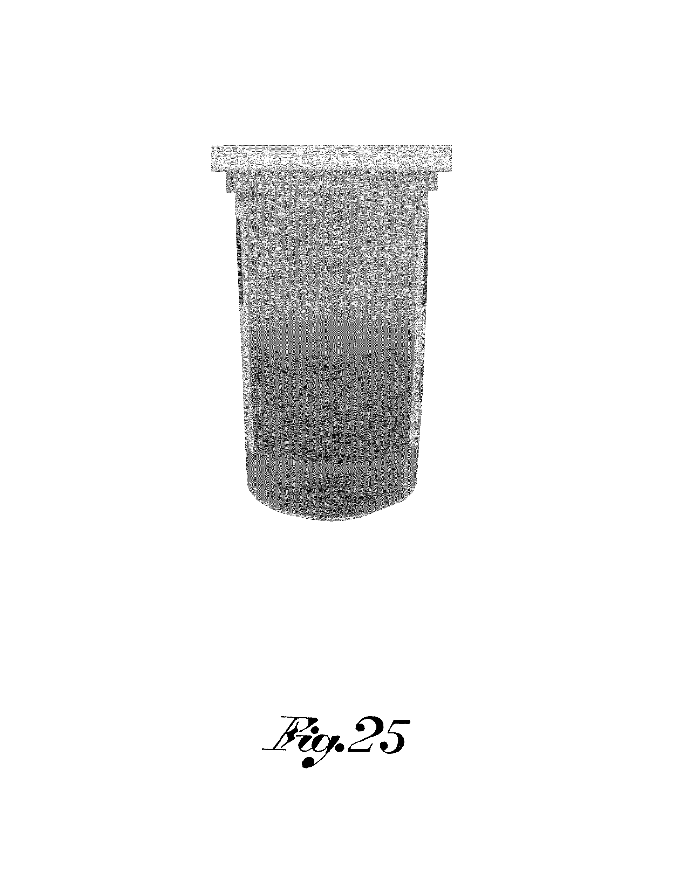

FIG. 25 is a view illustrating collected cells in an initial state, which start to be preserved in a supporting solution for cytodiagnosis of one example of the present invention. With reference to FIG. 25, materials, which can be collected from cervix using the brush of the present invention, include cervical cells, erythrocyte, and other mucous materials. Accordingly, other materials except for cervical cells must be removed by a proper method. In this example, in order to remove other materials except for required materials, i.e., cervical cells, the supporting solution for cytodiagnosis was maintained at a weak acidity. That is, since elasticities of membranes of by-products, such as erythrocyte, which was stuck to the bush or other tools when cervical cells are collected using the brush or other tools, are destroyed in a low acidity state, the by-products, such as erythrocyte, are dissolved in the supporting solution for cytodiagnosis of the present invention. On the other hand, a conventional cytodiagnosis requires a separate process for removing erythrocyte. Accordingly, the supporting solution for cytodiagnosis of the present invention solves the above drawback of the conventional cytodiagnosis.

In the examples of the present invention, each of the supporting solutions for cytodiagnosis included alcohol containing C2 to C4 for fixing the pathological structures of cells to be diagnosed. Further, each of the supporting solution for cytodiagnosis included an anti-desiccant, such as ethylene glycol, propylene glycol, glycerol, polyethylene glycol, or polypropylene glycol, for preventing stored cells from drying during various subsequent tests so that the cells retaining their original shapes are observed.

### [Morphologic Diagnostic Test according to Preservation time]

### 1. Test Object

Morphologic changes of cells, which are collected in the supporting solutions of cytodiagnosis manufactured in accordance with the various examples of the present invention, were observed according to preservation time of the cells. Thereby, cell-preserving capacities of the supporting solutions for cytodiagnosis of the examples were judged.

### 2. Test Method

Cells collected from cervix were preserved in the supporting solutions for cytodiagnosis of the examples of the present invention and the solutions of the comparative examples at room temperature. After 1 week, 1 month, 6 months, 12 months, and 18 months respectively elapsed, the preserved cells were attached to a polymeric filter membrane, and the cells attached to the polymeric filter membrane were transferred to glass slides. The cells on the glass slides were fixed in an ethanol solution of 95%, were stained with pap stain, and were then looked at under a microscope

### 3. Test Result

The cells, which were preserved in the supporting solutions for cytodiagnosis of the examples of the present invention, retained their original shapes even after approximately 12 to 18 months respectively elapsed, whereas the cells, which were preserved in the solutions of the comparative examples, did not retain their original shapes or had deformed shapes so that the cytological tests of the cells are impossible.

Table 1 states the above test results. With reference to Table 1, it is clear that the use of the anti-desiccant prolongs the cell preservation time.

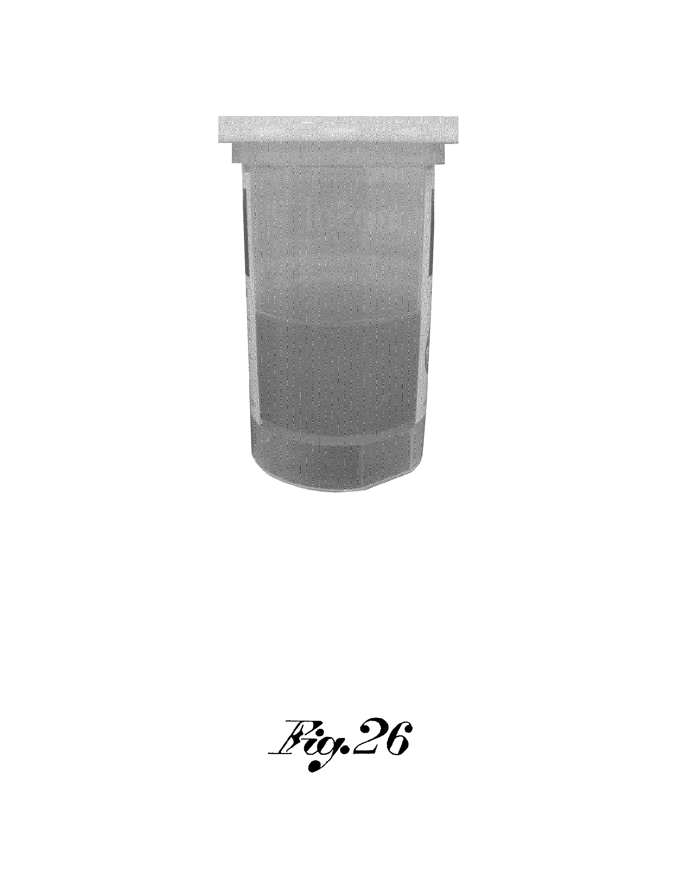

FIG. 26 is a view illustrating cells in a state, which have been preserved for at least 18 months in the supporting solution for cytodiagnosis of one example of the present invention.

### [Inflammability Test]

### 1. Test Object

Whether or not the supporting solutions for cytodiagnosis of the examples of the present invention are inflammable was judged.

### 2. Test Method

10cc of the supporting solutions for cytodiagnosis of the examples of the present invention and 10cc of the solutions of the comparative examples were contained in test vessels and were ignited with a lighter.

### 3. Test Result

The ethanol solution of 95% of the comparative example 1 suddenly ignited, but the supporting solutions for cytodiagnosis of the examples and the PBS solution of the comparative example 2 did not ignite. Table 1 states the above inflammability test results. With reference to Table 1, it is clear that the supporting solutions for cytodiagnosis of the examples of the present invention contain low alcohol content, are not inflammable, and are thus safe to use.

### [Erythrocyte Removal Rate Test and Staining]

### 1. Test Object

Whether or not erythrocyte stuck to the collected cells is removed by the supporting solutions for cytodiagnosis of the examples of the present invention was judged, and the cells preserved in the supporting solutions for cytodiagnosis of the examples of the present invention were stained and observed.

### 2. Test Method

### (1) Erythrocyte Removal Rate Test

A brush, having collected cervical cells, was rubbed onto the bottoms of the vessels containing the supporting solutions for cytodiagnosis of the examples of the present invention and the solutions of the comparative examples, thereby transferring the cervical cells stuck to the brush to the solutions. After approximately 1 day elapsed, 2cc of the solutions were collected from the respective vessels, and attached to polymeric filter membranes. Then, the solutions were transferred from the polymeric filter membranes to glass slides, were stained, and were then looked at under a microscope.

### (2) Staining

The brush, having collected cervical cells, was rubbed onto the bottoms of the vessels containing the supporting solutions for cytodiagnosis of the examples of the present invention and the solutions of the comparative examples, thereby transferring the cervical cells stuck to the brush to the solutions. After 1 month or 1 year elapsed, the supporting solutions and the solutions contained in the vessels were filtered out using polymeric filter membranes so that the preserved cells are transferred to the polymeric filter membranes. Thereafter, the cells were transferred from the polymeric filter membranes to glass slides. The cells on the glass slides were soaked in an ethanol solution of 95%, were stained with pap stain, and were then looked at under a microscope.

### 3. Test Result

### (1) Erythrocyte Removal Rate Test

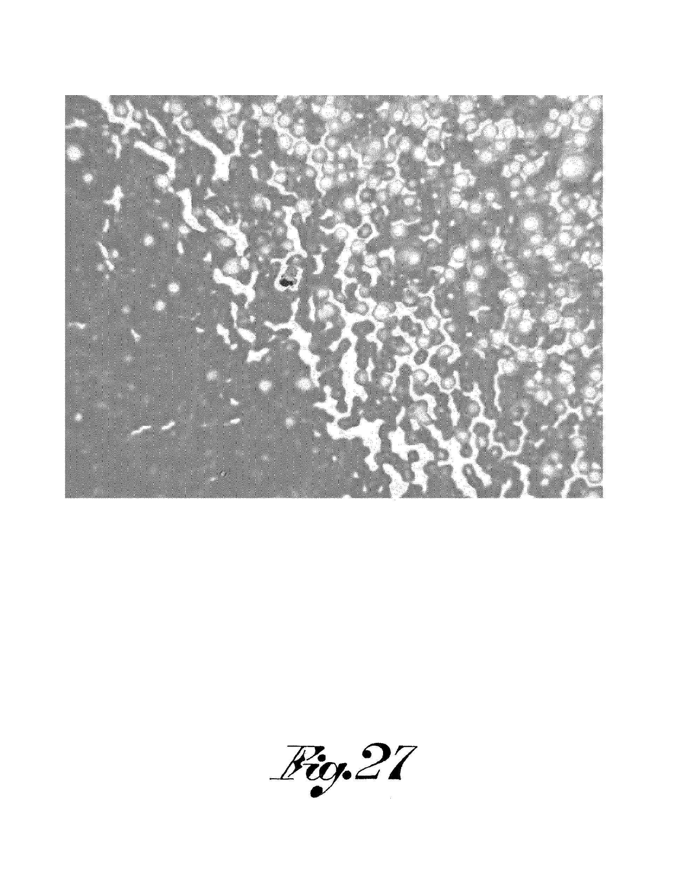

FIG. 27 is a microphotograph of the cells preserved in the ethanol solution of 95% of the comparative example 1. With reference to FIG. 27, erythrocyte was not removed from the cells preserved in the solutions of the comparative examples 1 and 2 during the preservation, but retains its original color, i.e., red, and their original shapes, i.e., disk shapes, and it was impossible to observe cervical cells with a microscope.

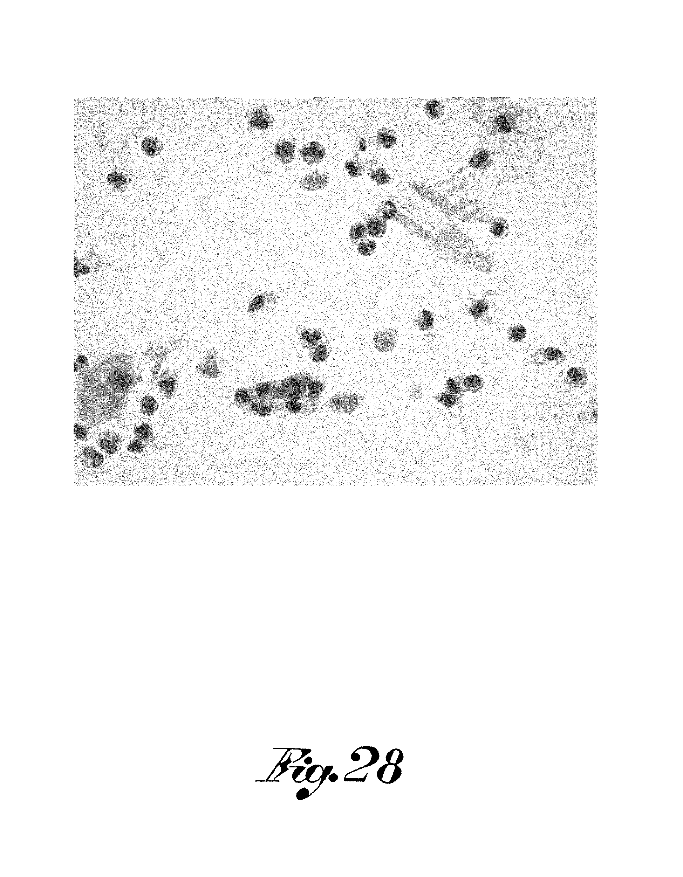

FIG. 28 is a microphotograph of cells preserved in the supporting solution for cytodiagnosis of one example of the present invention after 1 day elapsed. With reference in FIG. 28, erythrocyte was removed from the cells preserved in the supporting solution of the example, and is it possible to observe cervical cells with the microscope. The erythrocyte removal rates of the cells preserved in the supporting solutions of the examples of the present invention were approximately 85-95%, and are stated in Table 1 in detail.

### (2) Staining

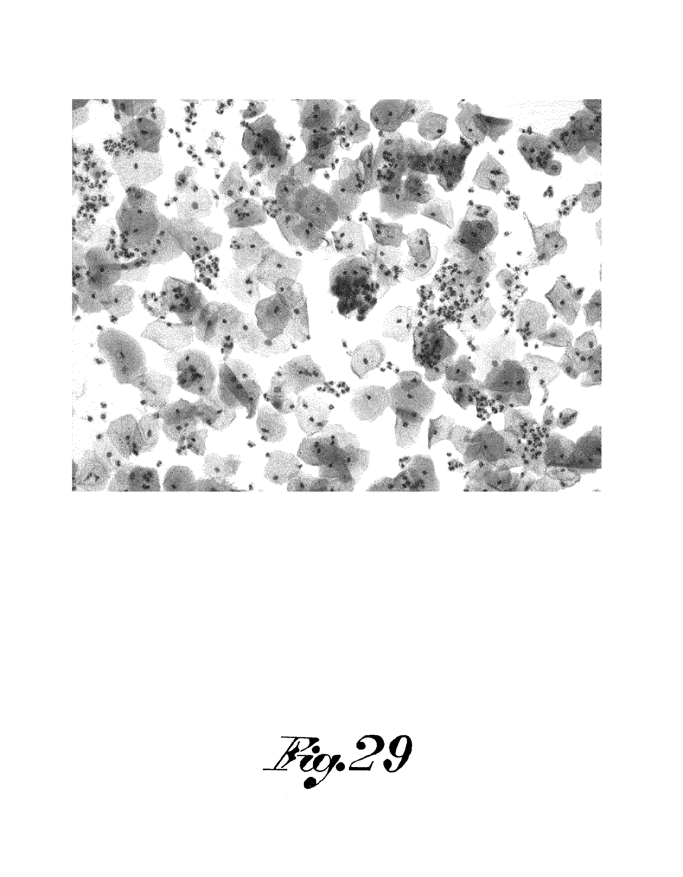

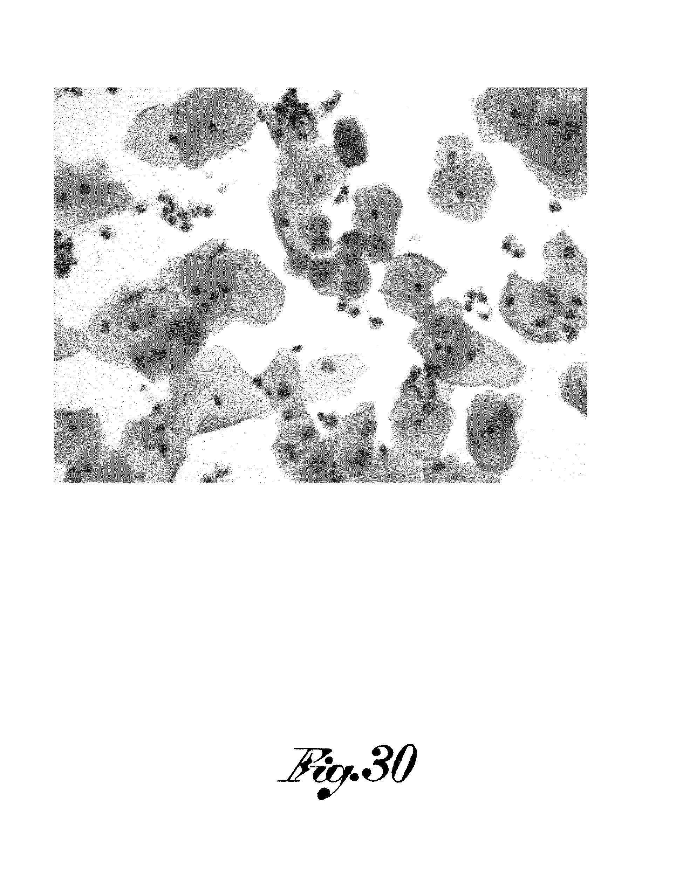

FIGS. 29 and 30 are photographs illustrating staining of cells of examples of the present invention. FIG. 29 illustrates staining of cervical cells, which were preserved in the supporting solution for cytodiagnosis of one example for 1 month. In this image, the cells were clearly observed, and divided into different colors according to characteristics of the cells (for example, normal cells or cancer cells). FIG. 30 illustrates staining of cervical cells, which were preserved in the supporting solution for cytodiagnosis of one example for 1 year. In this image, the cells were clearly observed, and divided into different colors according to characteristics of the cells. Accordingly, it was clear that the cells preserved in the supporting solutions of the examples of the present invention can be precisely diagnosed even after a long time elapsed.

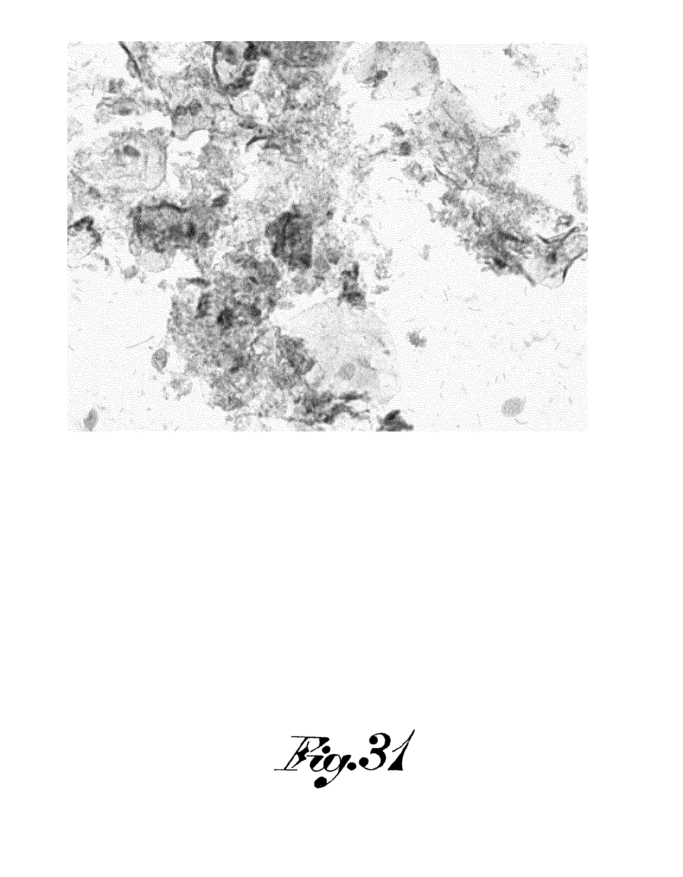

FIG. 31 is a photograph illustrating staining of cells preserved in a solution of one comparative example. In this image, the cells were denatured and destroyed or unclearly entangled, and cannot be precisely diagnosed.

### [Gene Analysis Possibility Examination

### 1. Examination Object

Cervical cells preserved in the supporting solutions for cytodiagnosis of the examples of the present invention were proliferated, and whether or not the gene analysis of the cells is possible was judged.

### 2. Examination Method

The cervical cells preserved in the supporting solutions for cytodiagnosis of the examples of the present invention were stored at room temperature for at least 1 month, and approximately 2cc of the supporting solutions were extracted. After Polymerase Chain Reactions (PCRs) of the extracted supporting solutions were repeated 25 or 30 times, electrophoretic photographs of the extracted supporting solutions were taken. The cervical cells preserved in the PBS solution of the comparative example 2 were stored by the same method for the same time, and approximately 2cc of the PBS solution was extracted. After PCR of the extracted PBS solution was repeated 25 or 30 times, an electrophoretic photograph of the extracted PBS solution was taken.

### 3. Examination Result

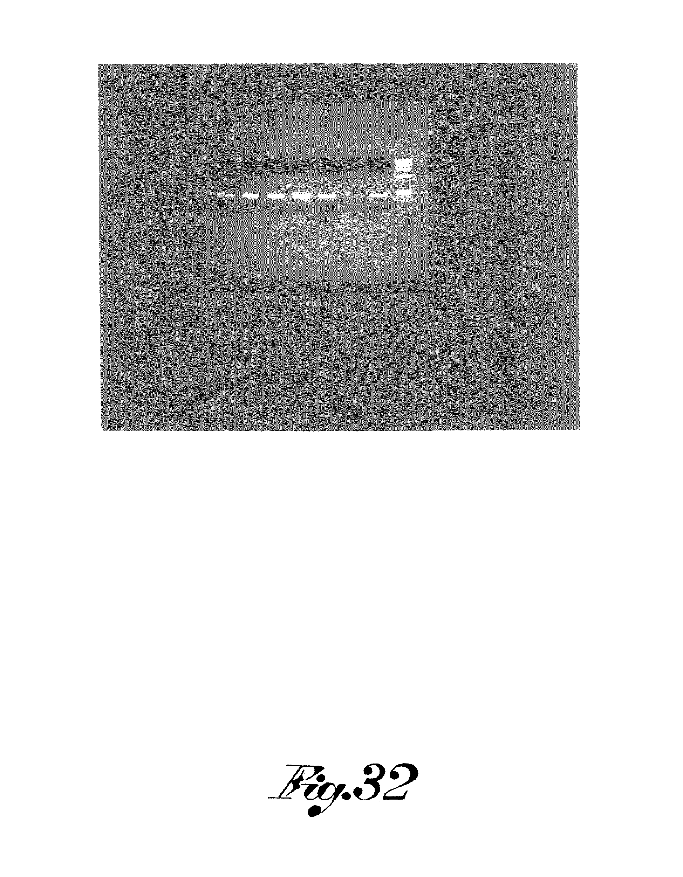

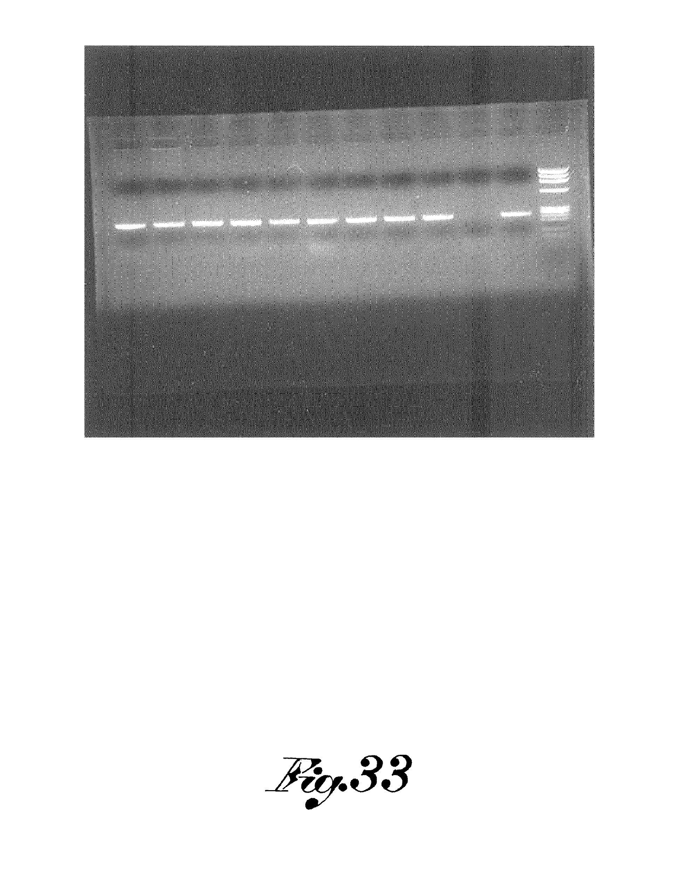

FIGS. 32 and 33 are electrophoretic photographs of cells preserved in the supporting solution for cytodiagnosis of the examples of the present invention. With reference to FIG. 32, the cells preserved in the supporting solution for cytodiagnosis of the example of the present invention can go through gene analysis by repeating the PCRs several times without any separate process. Further, the cells preserved in the supporting solution for cytodiagnosis of the example of the present invention at room temperature can go through gene analysis. The gene analysis rate of the cells preserved in the supporting solution for cytodiagnosis was approximately 99.9%. (In FIGS. 32 and 33, a negative mark denoted a negative control group.)

FIG. 33 is an electrophoretic photograph of cells preserved at room temperature for at least 1 year in the supporting solution for cytodiagnosis of one example of the present invention. As shown in FIG. 33, the cells preserved in the supporting solution for cytodiagnosis of the example of the present invention can go through gene analysis, even after 1 year elapsed.

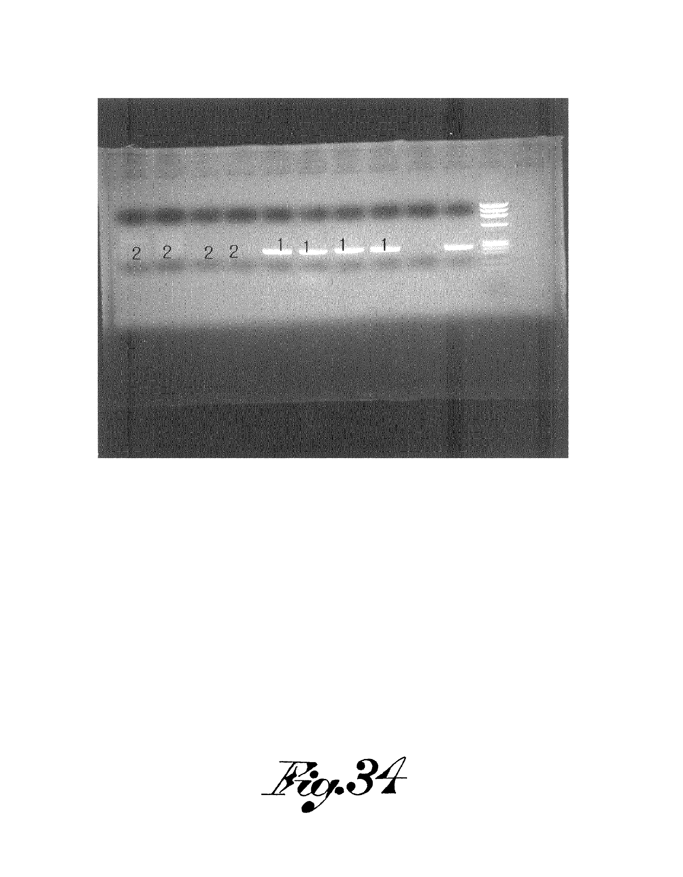

FIG. 34 is an electrophoretic photograph of cells preserved at room temperature for at least 1 month in the supporting solution for cytodiagnosis of one example of the present invention and in the PBS solution of one comparative example. With reference to FIG. 34, 1 denotes an electrophoresis result of the cells preserved at the room temperature in the supporting solution for cytodiagnosis of the example of the present invention, and 2 denotes an electrophoresis result of the cells preserved at the room temperature in the PBS solution of the comparative example. The cells preserved at the room temperature in the supporting solution for cytodiagnosis of the example of the present invention can go through gene analysis, whereas the cells at the room temperature in the PBS solution of the comparative example cannot go through gene analysis. When the supporting solutions for cytodiagnosis of the examples of the present invention are used to preserve cells, even though the cells are stored at room temperature, gene analyses of the cells is possible. Accordingly, compared to the conventional PBS solution, the supporting solutions for cytodiagnosis of the examples of the present invention facilitate gene analysis of cells.

Since the supporting solutions for cytodiagnosis of the examples of the present invention go through the gene analyses of the cells preserved in the supporting solutions without any separate process, the use of the supporting solutions for cytodiagnosis of the examples of the present invention causes rapid notification of results to patients, and allows cells, which are stored at room temperature for a long period of time, to go through gene analysis, thereby reducing costs to store the cells.

**[Table 1]**

| | Morphological diagnosis according to preservation time | | | | | Inflammability | Staining | Erythrocyte removal rate | Gene analysis |
|---|---|---|---|---|---|---|---|---|---|
| | 1 week | 1 month | 6 months | 12 months | 18 months | | | | |
| 1 | Proper | Proper | Proper | Proper | Improper | No | Proper | 85% | Possible |
| 2 | Proper | Proper | Proper | Proper | Proper | No | Proper | 90% | Possible |
| 3 | Proper | Proper | Proper | Proper | Proper | No | Proper | 90% | Possible |
| 4 | Proper | Proper | Proper | Proper | Proper | No | Proper | 90% | Possible |
| 5 | Proper | Proper | Proper | Proper | Improper | No | Proper | 90% | Possible |
| 6 | Proper | Proper | Proper | Proper | Proper | No | Proper | 95% | Possible |
| 7 | Proper | Proper | Proper | Proper | Proper | No | Proper | 90% | Possible |
| 8 | Proper | Proper | Proper | Proper | Proper | No | Proper | 90% | Possible |
| 9 | Proper | Proper | Proper | Proper | Proper | No | Proper | 85% | Possible |
| 10 | Proper | Proper | Proper | Proper | Proper | No | Proper | 95% | Possible |
| 11 | Proper | Proper | Proper | Proper | Proper | No | Proper | 95% | Possible |
| 12 | Proper | Proper | Proper | Proper | Proper | No | Proper | 95% | Possible |
| 13 | Proper | Proper | Proper | Proper | Improper | No | Proper | 95% | Possible |
| 14 | Proper | Proper | Proper | Proper | Proper | No | Proper | 95% | Possible |
| 15 | Proper | Proper | Proper | Proper | Proper | No | Proper | 90% | Possible |
| 16 | Proper | Proper | Proper | Proper | Proper | No | Proper | 90% | Possible |
| 17 | Proper | Proper | Proper | Proper | Proper | No | Proper | 90% | Possible |
| 18 | Proper | Proper | Proper | Proper | Proper | No | Proper | 90% | Possible |
| 19 | Proper | Proper | Proper | Proper | Improper | No | Proper | 90% | Possible |
| 20 | Proper | Proper | Proper | Proper | Proper | No | Proper | 90% | Possible |
| 21 | Proper | Proper | Proper | Proper | Proper | No | Proper | 85% | Possible |
| 22 | Proper | Proper | Proper | Proper | Proper | No | Proper | 90% | Possible |
| 23 | Proper | Proper | Proper | Proper | Proper | No | Proper | 90% | Possible |
| 24 | Proper | Proper | Proper | Proper | Proper | No | Proper | 90% | Possible |
| 25 | Proper | Proper | | Proper | Proper | No | Proper | 85% | Possible |
| 26 | Proper | Proper | Proper | Proper | Proper | No | Proper | 89% | Possible |
| 27 | Proper | Proper | Proper | Proper | Proper | No | Proper | 90% | Possible |
| 28 | Proper | Proper | Proper | Proper | Improper | No | Proper | 90% | Possible |
| 29 | Proper | Proper | Proper | Proper | Improper | No | Proper | 90% | Possible |
| Com. Example 1 | Proper | Improper | Improper | Improper | Improper | Yes | Improper for more than 1 week | Impossible | Partialypossible |
| Com. example 2 | Impossible when stored at room tem. | Impossible when stored at room tem. | Impossible when stored at room tem. | Impossible when stored at room tem. | Impossible when stored at room tem. | No | Improper | Impossible | Possible when stored in refrigerator |

### [Industrial Applicability]

The present invention is applied to a tumor screening system, a collection vial for liquid based cytology, brush for liquid based cytology of cervix carcinoma, and a supporting solution for cytodiagnosis.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A supporting solution for cytodiagnosis comprising:
a buffer agent for maintaining the acidity of the supporting solution in a designated pH range;
alcohol; and
an anti-desiccant for preventing the cells from drying during subsequent tests.

2. A supporting solution as set forth in claim 1, wherein the buffer agent maintains the acidity of the supporting solution at from pH 2 to 6.5.

3. A supporting solution as claimed in claim 2, wherein the buffer agent is one selected from the group consisting of lactic acid, citric acid, and phosphoric acid.

4. A supporting solution as claimed in claim 3, comprising 10 to 40 parts by weight of buffer agent.

5. A supporting solution as claimed in claim 1, wherein the alcohol is one selected from the group consisting of ethanol, propanol, isopropyl alcohol, butanol, and pentanol.

6. A supporting solution as claimed in claim 1, wherein the alcohol is one selected from the group consisting of ethanol, propanol, and isopropyl alcohol.

7. A supporting solution as claimed in claim 1, comprising 5 to 40 parts by weight of alcohol.

8. A supporting solution as claimed in claim 7, comprising 10 to 35 parts by weight of alcohol.

9. A supporting solution as claimed in claim 1, wherein the anti-desiccant is polyhydric alcohol.

10. A supporting solution as claimed in claim 9, wherein the polyhydric alcohol is one selected from the group consisting of ethylene glycol, propylene glycol, and glyceral.

11. A supporting solution as claimed in claim 1, wherein the anti-desiccant is polyethylene glycol or polypropylene glycol.

12. A supporting solution as claimed in claim 11, wherein polyethylene glycol or polypropylene glycol has a molecular weight of 100 to 100,000Da.

13. A supporting solution as claimed in claim 1, comprising 0.1 to 30 parts by weight of anti-desiccant.

14. A supporting solution as claimed in claim 13, comprising 0.1 to 15 parts by weight of anti-desiccant.

15. A supporting solution as claimed in claim 1, further comprising a preservative.

16. A supporting solution as claimed in claim 15, wherein the preservative comprises at least one selected from the group consisting of ascorbic acid, sorbic acid, citric acid, salicylic acid, sodium benzoate, formalin, glyoxal, and calcium propionate.

17. A supporting solution as claimed in claim 15, comprising 0.1 to 20 parts by weight of preservative.
